# EUROPEAN PATENT APPLICATION

(11) **EP 1 462 099 A1**
(43) Date of publication of application: **29.09.2004**
(21) Application number: 02790715.3
(22) Date of filing: 11.12.2002
(51) Int. Cl.: A61K 9/70, A61K 7/48, A61K 7/00

(54) **GEL COMPOSITIONS FOR EXTERNAL USE, PAD MATERIALS AND PAD MATERIALS TO BE PACKED IN BLISTER CONTAINERS**

(30) Priority: 13.12.2001 JP 2001380343; 10.01.2002 JP 2002000079; 10.01.2002 JP 2002003578; 08.03.2002 JP 2002064089; 03.04.2002 JP 2002101525; 29.07.2002 JP 2002219095
(71) Applicant: Dia Pharmaceutical Co., Ltd., Kashihara-shi, Nara 634-0803 (JP)
(72) Inventor: MORIKANE, Shinji, Kashihara-shi, Nara 634-0835 (JP); MORIKANE, Daizo, Kashihara-shi, Nara 634-0835 (JP)
(74) Representative: Sama, Daniele, Dr.
(86) International application number: PCT/JP2002/012948
(87) International publication number: WO 2003/053421

(57) **Abstract**

An object of the present invention is to provide a topical gel composition having high adaptability to human skins irrespective of the mode of use and having high strength. The present invention provides a topical gel composition formed of water soluble polymer having self-shape retainability, 30 to 95% by weight of water content, and 65 to 99% of a compressive deformation rate expressed by the maximum deformation rate till a partial fracture of the gel structure when the gel composition is compressed at a predetermined speed by using a plunger capable of pressing the gel composition at a constant speed.

## Description

### Technical Field

The present invention relates to a topical gel composition.

### Background Art

Heretofore, gelled products have been utilized as topical agents or topical base agents in the fields of medicines, cosmetics, quasi-drugs, daily and sanitary miscellaneous commodities.

For example, they are utilized by optionally mixing pharmaceutical ingredients, etc., in gelled products, shaping the gelled products to a predetermined shape with or without supporting on a non-woven fabrics into a sheet-like configuration capable of sticking on human skins.

However, since undulations or creases are present on human skins and, further, since they expand and contract in accordance with motions, they are poor in the adaptability to human skins such as being peeled easily. Further, if the sheet of the gelled products is made into a thin film so as to conform to undulations and creases to endure expansion and contraction, their strength becomes extremely fragile such as suffering from puncturing or tearing during use.

Further, gelled products having self-shape retainability have been formed into a sheet with no support of a non-woven fabric or the like and used as topical agents.

However, even when they have self-shape retainability, when the gelled products are used alone, since the gelled products per se are fragile, the gel structure suffers from fracture or flexion by pressure upon taking out from a vessel or a package. Further, when they are peeled from the surface of human skins, they are torn by the tensile force, residues remain on the surface of human skins, or partial residues may contaminate the skin surface.

For solving the foregoing subject, an earnest study has been made on the properties of the gelled products, particularly, on the strength and the flexibility thereof and, as a result, it has been found unexpectedly that a gel composition comprising a water soluble polymer capable of enduring the use as a topical agent or a topical base agent with respect to strength or flexibility and having a further good handlability can be obtained when it has a predetermined water content and a compressive deformation ratio to accomplish the present invention.

It has further been found that a pad material more conforming to the human skins and optimal to various application uses can be obtained by the use of such a gel composition to accomplish the present invention.

### Disclosure of the Invention

The present invention provides a topical gel composition, formed of a water soluble polymer, having a self-shape retainability, 30 to 95% by weight of a water content and 65 to 99% of a compression deformation rate that expresses a maximum deformation rate till partial fracture of a gel structure when the gel composition is compressed at a predetermined speed by using a plunger capable of pressing at a predetermined speed.

Further, it provides a pad material in which the topical gel composition has a sheet-like and a rectangular or substantially rectangular shape and a positioning part is formed at the center.

Further, it also provides a blister vessel-filled type pad material in which a solution of the topical gel composition is filled in a blister vessel having a predetermined shape and formed into a sheet-like shape corresponding to the shape of the blister vessel, and a blister vessel filled type pad material in which the topical gel composition is formed into a sheet-like shape, cut into a predetermined shape and contained in a blister vessel.

### Brief Explanation of the drawings

Fig. 1 shows a cooling sheet formed with a gel composition according to the present invention and contained in a portable type gel pack material as: (a) a perspective view, (b) a plan view for another cooling sheet, (c) and (d) a perspective view and a cross sectional view for a further cooling sheet, and (e) and (f) a perspective view and a cross sectional view for a still further cooling sheet.
Fig. 2 shows a face sheet formed with a gel composition of the invention as: (a) an upper plan view for an upper portion, (b) a front elevational view for upper and lower portions, (c) an upper view and a bottom view for the lower portion, (d) a bottom view for the upper portion, (e) a right or left side elevational view for the upper portion, and (f) a right or left side elevational view for the lower portion.
Fig. 3 shows another face sheet formed with a gel composition of the invention as: (a) an upper plan view for an upper portion, (b) a front elevational view for upper and lower portions, (c) an upper plan view and a bottom view for the lower portion, (d) a bottom view for the upper portion, (e) a right or left side elevational view for the upper portion, and (f) a right or left side elevational view for the lower portion.
Fig. 4 shows a still further face sheet formed with a gel composition of the invention as: (a) an upper plan view for an upper portion, (b) a front elevational view for upper and lower portions, (c) an upper plan view and a bottom view for the lower portion, (d) a bottom view for the upper portion, (e) a right or left side elevational view for the upper portion, and (f) a right or left side elevational view for the lower portion.
Fig. 5 shows a still further face sheet formed with a gel composition of the invention as: (a) an upper plan view for an upper portion, (b) a front elevational view for upper and lower portions, (c) an upper plan view and a bottom view for the lower portion, (d) a bottom view for the upper portion, (e) a right or left side elevational view for the upper portion, and (f) a right or left side elevational view for the lower portion.
Fig. 6 is a perspective view showing an eye pack formed with a gel composition according to the invention and contained in a portable type gel pack material.
Fig. 7 is (a) a plan view for a lip pack, (b) a cross sectional view and (c) a perspective view, formed with a gel composition according to the invention and contained in a portable type gel pack material.
Fig. 8 is a perspective view showing an another embodiment of a lip pack.
Fig. 9 is a plan view for a gel sheet formed of a gel composition according to the invention.
Fig. 10 is a plan view for a gel sheet formed with a gel composition according to the invention and an explanatory view showing the state of use of the gel sheet in which it is sticked from the vicinity of a neckline to an intermediate portion between right and left bladebones as the back.
Fig. 11 is an explanatory view of a gel sheet formed with a gel composition according to the invention in a state of use sticked on the back, the arm and the shoulder.
Fig. 12 is a perspective view showing an insect sting sheet formed with a gel composition according to the invention and contained in a portable type gel pack material.
Fig. 13 is a plan view of other cooling sheet formed with a gel composition according to the invention.
Fig. 14 is a plan view of another cooling sheet formed with a gel composition according to the invention.
Fig. 15 is a plan view of a further cooling sheet formed with a gel composition according to the invention.
Fig. 16 is a plan view of a further cooling sheet formed with a gel composition according to the invention.
Fig. 17 is a plan view of a further cooling sheet formed with a gel composition according to the invention.
Fig. 18 is a front elevational view in a case of sticking a cooling sheet formed with a gel composition according to the invention on a forehead.
Fig. 19 is a plan view of a further cooling sheet formed with a gel composition according to the invention.
Fig. 20 is a schematic plan view and a cross sectional view of a pad material formed with a gel composition according to the invention.
Fig. 21 is a cross sectional view showing the state of dispersing fibers inside the pad material formed with a gel composition according to the invention.
Fig. 22 is a cross sectional view showing the state of providing an air permeable sheet to a pad material formed with a gel composition according to the invention.
Fig. 23 is a cross sectional view showing a state of providing an air permeable sheet to a pad material formed with a gel composition according to the invention and forming apertures corresponding to through holes of the pad material.
Fig. 24 is a schematic plan view of a vessel containing a pad material.
Fig. 25 is a schematic view showing a state of containing the pad material in a containment concave part of a vessel and tightly sealing the same by an airtight film.
Fig. 26 is schematic view showing a state of accommodating in a bag an entire vessel containing the pad material in the containment concave part and sealing the same.
Fig. 27 is schematic plan view and cross sectional view of a pad material formed with a gel composition according to the invention.
Fig. 28 is a cross sectional view for explaining the cross sectional shape of a heat dissipating concave part of a pad material formed with a gel composition according to the invention.
Fig. 29 is a schematic cross sectional view showing a state of containing a pad material formed with a gel composition according to the invention in a containment concave part of a vessel and tightly sealing the same with an air tight film.
Fig. 30 is schematic plan view and explanatory cross sectional view of a pad material formed with a gel composition according to the invention.
Fig. 31 is a cross sectional view showing a state of forming heat dissipating grooves continuously to a pad material formed with a gel composition according to the invention.
Fig. 32 is schematic plan view and cross sectional view of a pad material formed with a gel composition according to the invention.
Fig. 33 is a schematic cross sectional view of a pad material formed with a gel composition according to the invention.
Fig. 34 is a schematic perspective view of a vessel for containing a pad material formed with a gel composition according to the present invention.
Fig. 35 is a schematic cross sectional view showing a state of containing a pad material formed with a gel composition according to the invention in a containment concave part of a vessel and tightly sealing the same with an air tight film.
Fig. 36 is a schematic cross sectional view showing a state of providing a pad take-out member to a pad material formed with a gel composition according to the invention.
Fig. 37 is a schematic cross sectional view of a pad material provided with a thin portion inclined moderately at the periphery of an sticked surface and provided with a reinforcing rib to the thinned portion inclined moderately to the pad material formed with a gel composition according to the invention.
Fig. 38 is a schematic cross sectional view of a pad material formed with a gel composition according to the invention.
Fig. 39 is a schematic perspective view for a vessel for containing a pad material and a lid material.
Fig. 40 is a schematic cross sectional view showing each of modified examples for the adsorbing concave part provided to the pad material formed with a gel composition according to the invention.
Fig. 41 is a schematic plan view showing each of modified examples of a vessel in which a protrusion corresponding to the adsorbing concave part provided to the pad material is provided at the bottom of the containment concave part.

### Best Mode for Carrying Out the Invention

The topical gel composition according to the invention mainly comprises a water soluble polymer.

The water soluble polymer has no particular restriction for the kind thereof so long as it shows the following water content and the compressive deformation rate when formed into a gel composition. Further, those which are transparent when prepared as a gel composition are preferred. Transparency means herein a state of colorless with no clouding which is so-called as water clear. The solubility of the water soluble polymer means such a property that 1 g of the water soluble polymer can be dissolved in about less than 30 ml of water at about room temperature.

For the water soluble polymer, natural water soluble polymer, particularly, polysaccharides are preferred. In a case of the natural water soluble polymers, those having a gel strength, for example, of about 60 to 100 g are suitable. The gel strength means herein a strength expressing, by the number of grams, a force necessary for pressing the surface of a gelled product of a water soluble polymer 4 mm down by a plunger at a rate of 6 cm/min. by using a rheometer, for example, having a plunger of 0.5 inch. Specific examples of natural water soluble polymers can include, for example, carrageenan, locust bean gum; xanthane gum; guar gum; gellan gum; tamarind seed gum; caraya gum; pectin; glucomannan, gelatin, etc.

Further, synthetic water soluble polymer may also be used and the average molecular weight in this case can include, for example, of about 1000 to 200,000,000. Specifically, they can include carboxy methyl cellulose, sodium carboxy methyl cellulose; carboxyvinyl polymer; polyvinyl alcohol, alginic acid, graft starch, agarose, polyglutamic acid, polyacrylic acid, alginic acid, honest gum or salts thereof and partially neutralized products thereof.

The water soluble polymer may be used alone or as a mixture of two or more of them. Among them, combination of two or more of them is preferred and use of natural water soluble polymers in combination of two or more of them is more preferred. Particularly, it is preferred that carrageenan is contained. Carrageenan is generally extracted from sea weed referred to Irish moss, which is a sort of a polysaccharides called as galactan containing sulfate groups which include κ, 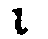 and λ types. In the present invention, any of them may be used, however, κ and/or 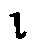 types may be preferably used. In a case of using carrageenan, it is preferred that both of the κ type and the 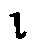 type are contained and the mixing ratio of carrageenan with other water soluble polymer is preferably, for example, about 1:0 (weight ratio) to 1:10 (weight ratio). Further, in a case of using both of the κ type and the 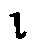 type, it is preferred that κ type: 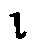 type = about 1:10 (weight ratio) to 10:1 (weight ratio). A further preferred specific combination is that of κ type and/or 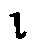 type carrageenan: locust bean gum: glucomannan, and the ratio of combination is about 10 to 1: 10 to 1:1 (weight ratio) although not restricted particularly.

The water soluble polymer is appropriately contained by about 0.1 to 10 % by weight based on the entire gel composition. It is preferably from 0.3 to 5% by weight, and more preferably about 0.5 to 3% by weight. The water soluble polymer is preferably edible. "Edible" means herein that drinking or eating is authorized by food regulating laws.

Further, the topical gel composition is formed by dissolving the water soluble polymer described above in water and gelling the same and has a water content of about 30 to 95%, preferably about from 40% or more, 50% or more, 90% or less, 85% or less and 80% or less. In a case where the water content is less than about 30%, it results in disadvantage such that the compressive deformation rate described later can not be obtained, water retainability is remarkably lowered, it tends to be defoliated from skins, results in drying of skin surface and skin eruptions. Further, if the water content exceeds 95%, the compressive deformation rate lowers remarkably making the gel composition brittle and no more sufficiently endurable to use, it shows remarkable release of water during use, worsens the handlability and tends to be defoliated from the skins as well.

Further, the compressive deformation rate of the topical gel composition is about from 65 to 99%, preferably, about 70% or more, 75% or more, 98.5% or less, 98% or less, 97% or less, 95% or less, 92% or less and 90% or less.

The compressive deformation rate is a maximum deformation rate, represented by percentage, till a gel structure reaches a partial fracture when a gel composition is compressed at a predetermined speed by using a plunger capable of pressing at a predetermined speed. More specifically, when the compressive deformation rate is measured, the gel composition is preferably a plate-like specimen of a predetermined thickness. Further, the plunger preferably has predetermined cross sectional area and such a plunger is generally assembled in a compression tester. Accordingly, the compressive deformation rate in the present invention can be measured by a compression tester. The specimen upon measurement is, appropriately, in a plate shape having, for example, a thickness of about 1 to 10 mm and, further, about 2 to 4 mm. As the compression tester, a texture meter for measuring feeling upon eating or a rheometer for measuring viscoelasticity can be usually used. The plunger preferably has a cross sectional area, for example, of about 0.1 to 2 cm² and, further, a cross sectional area of about 0.5 cm², and the shape is preferably of a substantially cylindrical shape. Further, the speed of the plunger is about from 0.01 to 0.1 mm/sec. and about at 0.05 mm/sec. Within the range of the compressive deformation rate, physical properties such as desired tensile strength and adaptability to predetermined undulations or creases can be attained, to obtain a gel composition of excellent handlability.

The gel composition according to the present invention can be obtained by dissolving a water soluble polymer in water in a range of temperature about from 60 to 90°C and then allowed the solution to cool to about 20 to 40°C thereby gelling the same. In this case, medicine (for example, anti-inflammatory agent, analgesic agent, itch killing agent, vitamin, etc.) or cosmetics (astringent, collagen, etc.) may be added as active ingredients, and additives used in the field of medicines, cosmetics and foodstuffs, for example, antiseptic agent, sterilizer, fragrances, colorants, anti-oxidants etc. may be added. Further, aroma oils, etc. used in aroma therapy may also be added.

Medicines can include, for example, menthol, camphor, indomethacine, ketoprophene, dichlorophenac, phellodendron bark extract, dipotassium glycyrrhizinate, hyaluronic acid, arbutin, tocopherol acetate, allantoin, vitamin C, water soluble azulene, acrinol, etc. These medicines can be used as granules coated with polymer film, or may be used being impregnated into and carried on porous polymer granules.

As aroma oils, essential oil ingredients (essential oils) generally used for aroma therapy may be used as they are. The essential oil ingredients can include those described, for example, in "84 Essential Oil Ingredients for Aroma Therapy", written by Wander Celler, published from Fragrance Journal Co. Specifically, they can include those extracts, for example, from anise, angelica, benzoin, immortelle, chamomile, garlic, cardamon, galbanum, caraway, carrot seed, guaiac wood oil, grapefruit, cypress, cedar wood, star anise, sage, geranium, celery, thyme, estragon, turpentine, olibanum, violet, pine, parsely, fennel, black pepper, small leaved linden flower, lemon, lemongrass, rosemary, laurel leaf, meadowsweet, Japanese spirea, cornflower, almond, arnica, fennel, scotch broom, watercress, gentiana, camphor, plum, poire, Zizyphi fructus, dandelion, anemarrhenae, polyporus seed of wax gourd, rosa multiflora, peppermint, Fraxinus japonica, hoelen, merissa, peach, parasite, eucalyptus, coix seed, lavender, forsythia, etc.

As cosmetics and additives, those known in relevant field can be used.

The gel composition according to the invention can be used in the field of medicines, cosmetics, quasi-drugs, daily or sanitary miscellaneous commodities being sticked or in contact with human skins. Accordingly, the gel composition can be formed in the shape according to the application uses thereof. Specifically, they can include cooling sheets of an appropriate size for applying on foreheads, arms, legs, back, waist, joints and shoulders in the form of a sheet with a thickness of about 0.5 to 5 mm, a circular or elliptic face mask covering the entire face with a thickness of about 0.5 to 5 mm, a crescent-shaped or rectangular eye pack or lip pack with a thickness of about 0.5 to 5 mm, circular or rectangular itch-killing patch or pimple patch with a thickness of about 0.5 to 5 mm, gel sheet of various shapes for burn/sunburn/glowing with a thickness of about 0.5 to 5 mm, rectangular or cuboidal square cylinder or circular cylinder gel cake substrate.

The topical agents can be used as a gel composition not containing active ingredients or as a topical substrate containing active ingredients such as medicines or cosmetics therein. Further, they can be used together with a support such as non-woven fabric or may be used alone without using a support since they have self-shape retainability. In a case of forming the gel composition as a sheet-like, the cross sectional shape thereof may include various shapes such as elliptic, square, trapezoidal or inverted trapezoidal shape. Among them, a trapezoidal shape is preferred since peeling from skins can be prevented effectively when a surface with a large area in view of plane is sticked on human skins.

Particularly, the gel composition according to the present invention is used preferably as a pad material. While the pad material may be in various shapes depending on the application uses as described above, it preferably has, for example, a rectangular or substantially rectangular shape. The rectangular shape means herein a so-called laterally elongate shape in which comers thereof are not necessarily orthogonal but they may be rounded. This includes any shape in which the rectangular shape deforms while keeping its laterally elongate shape.

The pad material is preferably formed with a positioning part at the center thereof. The positioning part can include, for example, a concave recess or extension formed at the center of an upper edge or lower edge, or concave part, convex part, or a perforation formed on a vertical center line on the surface or the rear face.

The concave recess or extension has no particular restriction but it is preferably in such a shape that positioning is easy at the central portion of the pad material although not covering boarders of hair at the forehead, and the upper edge is preferably formed with a concave recess. Further, an extension is preferably formed to the lower edge for easy positioning by aligning with a portion between eyebrows although not covering eyebrows. The concave recess or the extension has, for example, a width of about 10 to 80 mm, preferably, about 10 to 50 mm, a depth or height of about 5 to 20 mm, preferably, 5 to 10 mm. Further, the concave recess or the extension is preferably formed with a curve, particularly, with a moderate curve so as to prevent tearing starting from the vicinity thereof.

The shape for the concave and convex parts has no particular restriction but it is preferably a shape easy for positioning. For example, it is preferred that the concave or convex part is formed to a height (depth) of about 1 to 3 mm and with a size of about 1 to 200 mm² to either the surface or the rear face not in contact with human skins. In a case of a through hole, the size is preferably about 1 to 200 mm².

The planar shape of the pad material is preferably an appropriate shape for applying, for example, to a face or a forehead as a cooling sheet. Specifically, it includes a square or corner-rounded square shape, or an elliptic or modified elliptic shape with the length for the longest vertical portion of about 40 to 90 mm and the length for the longest lateral portion of about 100 to 200 mm including the concave recess or extension to be described later.

Further, the pad material according to the present invention is preferably formed with a notch extending from the edge to the inside. The notch may be in any shape, size and number so long as it can deform the planar shape of the pad material corresponding to the shape of the application portion. For example, it is preferably in such a shape, size and number that can apply the pad material to an appropriate portion by pulling up or down the upper end of the pad material conforming to the shape of the boarder of hair on both ends of the forehead, the size of the forehead, etc. Specifically, it is preferably a slit-like shape or substantially trigonal shape with the width (maximum width) of about 1 to 5 mm and the length of about 10 to 40 mm. The position is appropriately at the peripheral edge of the sheet-like gelled product, for example, about 1/3 to 2/3 from top of the upper edge or both side edges. The notch may be arranged vertically to the side edge or the upper edge, or may be arranged being inclined at an angle of 30 to 90°. The number of the notches has no particular restriction and they may be formed by plurality to the edge and/or upper end but preferably formed each by one or two.

Further, the pad material according to the invention is preferably provided with a heat dissipation promoting portion for heat dissipation with an area of contact with external air being increased. For example, it is possible to form a heat dissipating through hole extending from the sticked surface to the opposite surface to form a heat dissipating concave part or groove on the surface opposite to the sticked surface, or to form a heat dissipating channel at an appropriate position in the thickness of the pad material.

Further, in the pad material according to the invention, for smooth movement of liquid at the inside, for effective evaporation of moisture content in the pad material through the heat dissipation promoting portion for heat dissipation, or for reinforcing the pad material per se, fibers which are hydrophilic at least on the surface are preferably dispersed inside the pad material. The fibers include, for example, pulp, fine fibrous cellulose, cotton threads, rayon and like other various hydrophilic resin fibers, as well as various hydrophobic resin fibers applied with hydrophilic treatment on the surface, glass fibers and ceramic fibers. The fibers are preferably dispersed such that they are entangled three dimensionally inside the pad material and exposed to the surface of the heat dissipating promotion portion for conducting heat dissipation by increasing the area of contact with external air.

Further, in the pad material according to the present invention, an adsorption concave part is preferably formed on the surface as an sticked surface or rear face. The concave part is evacuated in a case of sticking the pad material under pressing to the applying portion of a human body and sucked to the sticking portion. Even in a case that the amount of the gel composition is increased or the adhesion on the sticking surface is low, the pad material can be sticking stably to an sticked portion by means of the concave part.

Further, the pad material according to the invention is preferably formed such that the thickness at least a portion in the periphery is reduced toward the end edge. Thus, the pad material can be sticked along the applying portion which increases the ratio of area of the sticked surface relative to the amount of the gel composition and the pad material can be sticked stably to the applying portion even in a case of increasing the amount of the gel composition or in a case where the adhesiveness of the sticked surface is low. Particularly, when the thickness is reduced toward the side edge of the sticked surface for the entire periphery of the pad material, the pad material can be sticked more stably to the applying portion. Also, at least a portion in the periphery of the sticked surface may be reduced to the end edge of the surface opposite to the sticked surface. In the case of reducing the thickness toward the end edge, it is preferably reduced linearly or in a concave profile. Particularly, in a case of reducing the thickness in a concave profile, the pad material can be sticked to the applying portion more stably. In this case, since the strength is lowered on the end edge where the thickness is reduced, a reinforcing rib may be disposed to the end edge or the vicinity thereof on the surface opposite to the sticked surface.

In the pad material according to the invention, a support may be disposed to the surface or the rear face. The support has a size identical with or larger than that of the gelled pad. In a case of enlarging the support, it can be adhered to the applying portion by turning the support around the applying portion and engaging the ends of the support to each other, or by disposing a fixing adhesive layer to the outer edge of the support. Accordingly, even in a case of increasing the amount of the gel composition, the pad material can be fixed reliably to the applying portion by the support.

The pad material per se may also have a shape configurating the face or a body of an animal or a character so long as it does not impair each of the functions as will be described later.

The gel composition (or pad material) can be supplied being packed as an appropriate size or amount in a polyethylene bag or the like or in a blister package. In a case of containment in the blister package or the like, the blister package itself may be utilized for the molding of the gel composition, or a gel composition is cast into a predetermined shape to mold into a sheet-like shape, cut-out or punched into a desired shape, which may be contained in the blister vessel. In a case of molding and containment by using the blister package, etc., concave/convex portions may be preferably formed in the two-dimensional direction like a mesh-like shape to a region of the blister package other than the concave part in which the gel composition is to be contained. Thus, even in a case where the gel composition is formed into a large sheet-like shape, a flat sheet with no strain or distortion can be obtained.

Further, mesh-like shape or fine dot-shaped concave/convex pattern are preferably formed to the bottom of the concave part of the blister package. Then, upon molding the gel composition in the concave part, the concave/convex pattern develops to the surface of the gel sheet to cause random reflection making bubbles, etc. intruding into the gel composition not conspicuous. Further, a concave/convex part configurating the face or the body of an animal or the face or the body of a character, specifically, the face or the body of a dog, cat, pig, bear, elephant, etc., and the face or the entire body of a character such as Sazae-san, Doraemon, Anpanman, Astro Boy, etc., may be disposed for the concave part. This enables to obtain a pad material configurating an animal or a character by using the blister package for the pad material. Further, in a case of forming the blister vessel-filled type pad material containing a gel composition in a blister vessel, a support may be located on the surface or the rearface of the gel to be contained.

As the material for the blister vessel, a thermoplastic resin capable of injection molding, vacuum molding or pressure forming (for example, polypropylene, polyethylene terephthalate and polystyrene) is used preferably with no restriction to them. Further, material having light permeable or light screening property is preferably used in view of the property of the gel composition to be contained or various substances added to the gel composition. The blister vessel may be constituted with a single kind of resin, a lamination product of plural resin layers, or a lamination product of a resin layer with a metal layer or other layer. The thickness of the blister vessel is preferably set so as to have a mechanical strength endurable to the force exerted during wearing distribution and storage and it is for example, at 30 to 1000 µm in a case of using injection molding.

The pad material according to the present invention may be formed by using the specified gel composition according to the invention so as to have one or more features that (1) a positioning part is formed at the center, (2) a notch capable of deforming the planar shape is formed, (3) a heat dissipation promoting portion is formed, (4) an adsorption concave part is formed, (5) the thickness is reduced toward the end edge, and (6) the shape has a concave recess. So long as one or two of the features in view of the shape described above is provided, any gel composition may be used not restricted only to the specified gel composition described above. That is, it may be any of (1) a pad material in which the gel composition has a sheet-like and rectangular or substantially rectangular shape and a positioning apart is formed at the center, (2) a pad material in which the gel composition has a sheet-like or rectangular or substantially rectangular shape, and a notch extending from the edge to the inside and capable of deforming the planar shape conforming the shape of the applied portion, (3) a pad material in which the gel composition has a sheet-like and rectangular or substantially rectangular shape and a heat dissipation promoting portion for increasing the area of contact with external area and conducting heat dissipation is formed on the surface or the rear face, (4) a pad material in which the gel composition has a sheet-shape and a rectangular or substantially rectangular shape and an adsorbing concave part is formed to the surface or the rear face, (5) a pad material in which the gel composition is a sheet-like shape and the thickness for at least a portion in the periphery is reduced toward the end edge, or (6) a pad material in which the gel composition has a sheet-like shape and at least a portion of the peripheral edge has a concave recess curved smoothly to the inside and enhancing the adhesion of the entire peripheral edge of the sheet to the cured surface upon sticked to the curved surface.

Description is to be made to the examples of the topical gel composition, the pad material and the blister vessel-filled type pad material according to the invention.

### Preparation of gel composition

Gel compositions were prepared according to the following prescriptions with respect to the total amount (100% by weight) of each gel composition.

### Example 1

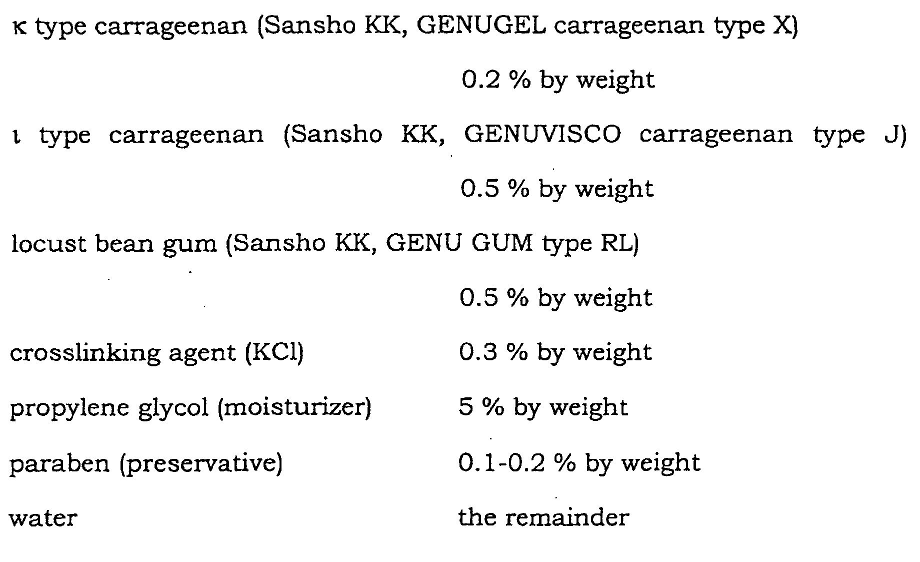

### Example 2

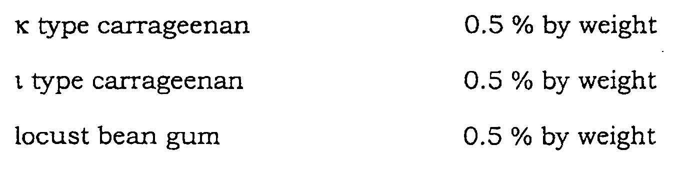

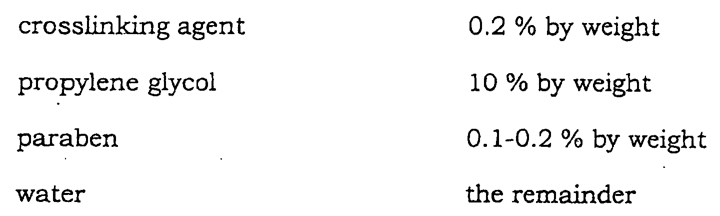

### Example 3

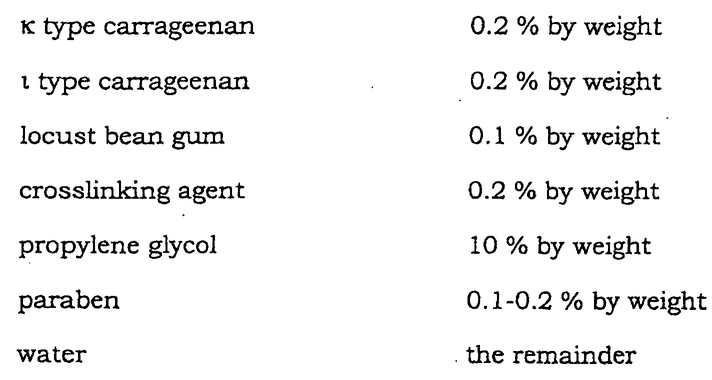

### Example 4

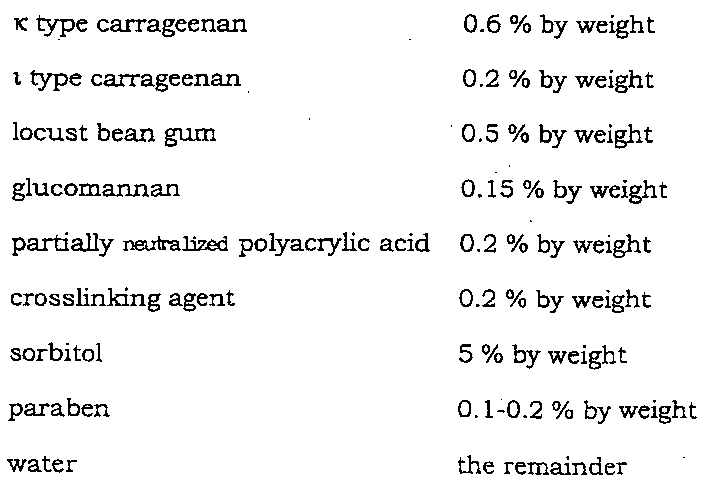

### Example 5

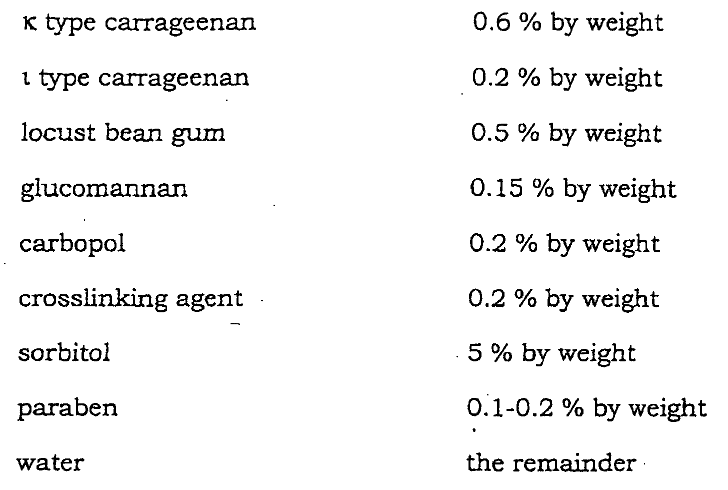

### Example 6

| | |
|---|---|
| κ type carrageenan | 0.6 % by weight |
| glucomannan | 0.4 % by weight |
| sorbitol | 5 % by weight |
| paraben | 0.1-0.2 % by weight |
| water | the remainder |

### Example 7

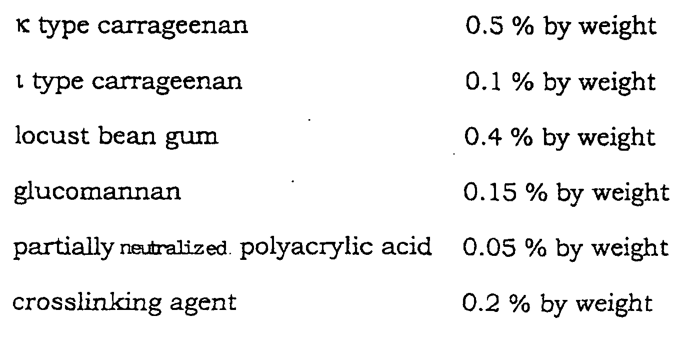

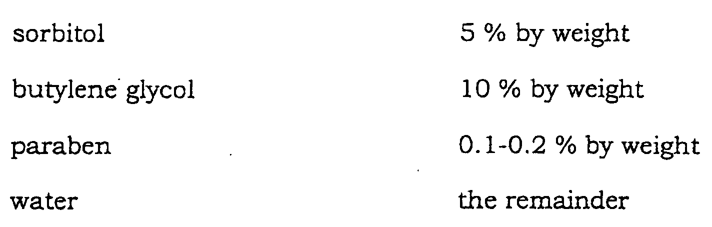

### Example 8

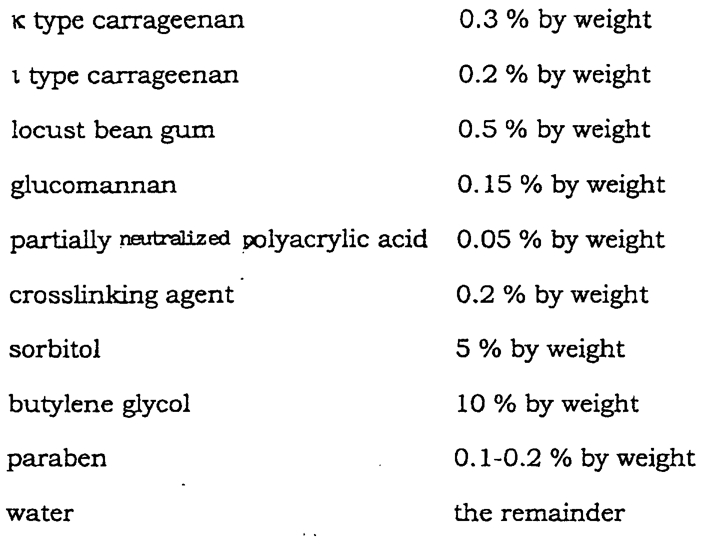

### Example 9

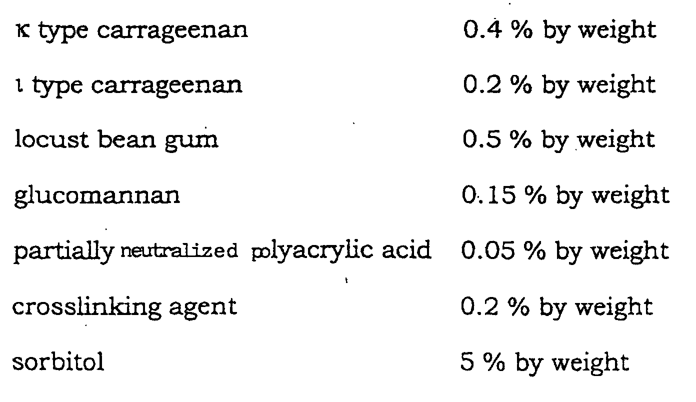

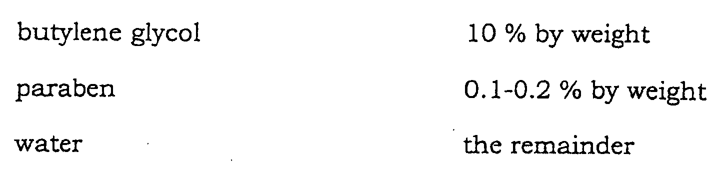

### Example 10

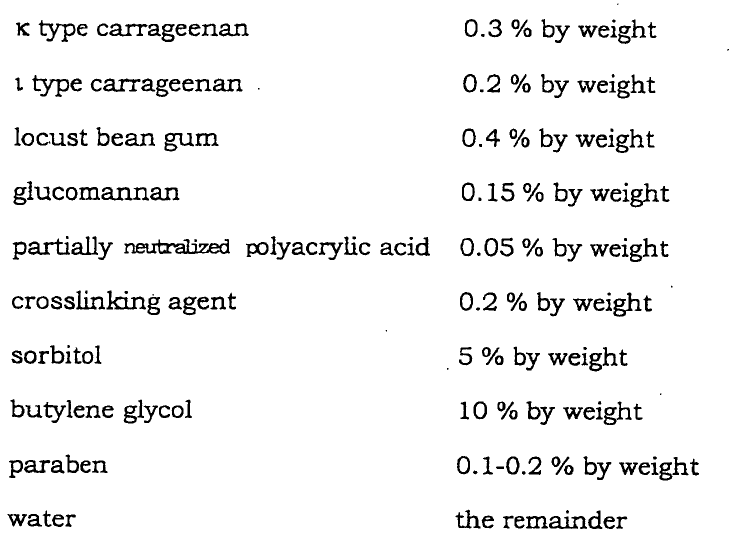

### Example 11

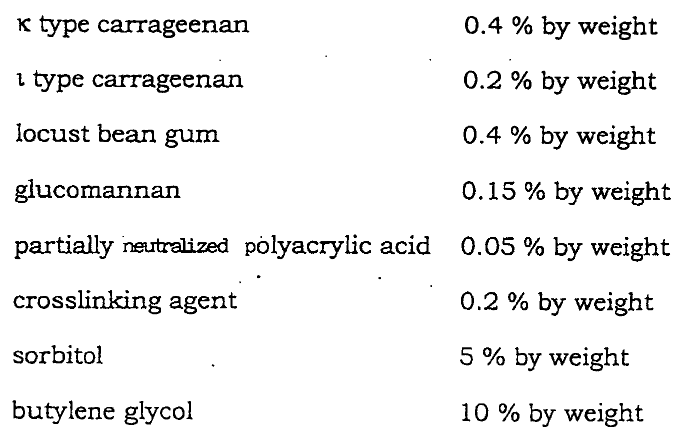

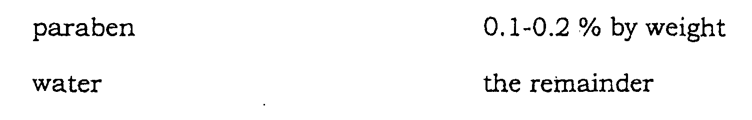

### Example 12

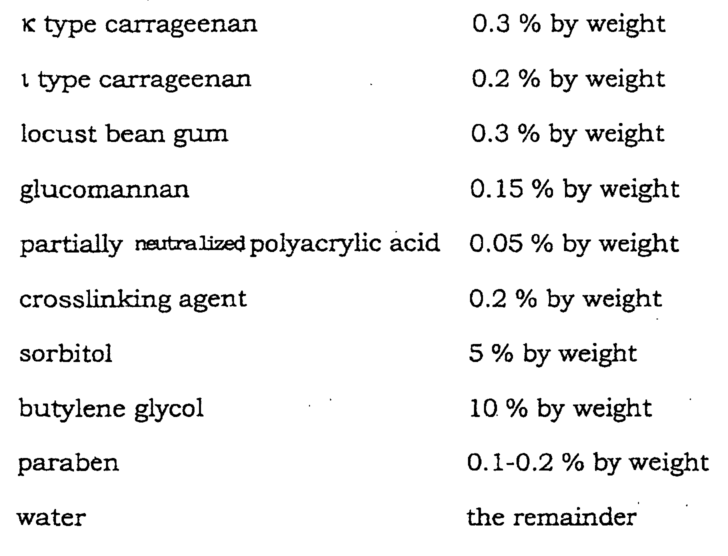

In the above Examples 1-12, the common method for preparing each gel composition was as follows.

That is, a small amount of water was put in a vessel and a crosslinking agent was added to the water and dissolved therein. On the other hand, gel component such as κ-type carrageenan, 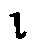 -type carragreenan, locust bean gum, glucomannan, partially. neutralized polyacrylic acid, etc. were added little by little to about a half amount of butylene glycol with stirring to obtain a liquid containing the gel components. (The gel components may be previously wetted and dispersed in a small amount of ethanol). Paraben was added to the remaining butylene glycol and completely dissolved with stirring under warming to give a solution. After cooling, the solution was added little by little to the above-mentioned liquid of the gel components with stirring.

Then, the mixture was added to the remaining water with stirring (in case where sorbitol is contained, sorbitol was also added thereto), and the gel components were swollen. The resultant swollen liquid of the gel components was heated to about 80-85°C. When the swollen gel components were homogeneously dissolved, the above aqueous solution of the crosslinking agent was added dropwise thereto, and the mixture was kept at 80-85°C for about 30 minutes. Then, the mixture was cooled in a water bath controlled at 70-75°C until the bubbles in the liquid were removed. Thus obtained hot solution for forming the gel composition was poured into a predetermined mold and left to stand at room temperature or cooled to room temperature to give a formed gel.

### Test Example 1

Compressive deformation rates of 12 kinds of the gel compositions obtained in the above Examples were measured. The measurement of the compressive deformation rate was carried out as follows. That is, a gel composition was compressed at a speed of 0.05 mm/sec. by using a compression tester, Texograph (Nihon Shokuhin Kenkyusyo) equipped with a cylindrical plunger having a cross sectional area of 0.5 cm², and the compressive deformation rate just before the partial fracture of the gel composition was determined. The compressive deformation rate of a gel composition is an average value obtained from five samples.
Composition of Example 1: Compressive deformation rate 81.5 %
Composition of Example 2: Compressive deformation rate 90.3 %
Composition of Example 3: Compressive deformation rate 75.3 %
Composition of Example 4: Compressive deformation rate 86.7 %
Composition of Example 5: Compressive deformation rate 90.8 %
Composition of Example 6: Compressive deformation rate 97.3 %
Composition of Example 7: Compressive deformation rate 91.5 %
Composition of Example 8: Compressive deformation rate 95.9 %
Composition of Example 9: Compressive deformation rate 97.6 %
Composition of Example 10: Compressive deformation rate 98.1 %
Composition of Example 11: Compressive deformation rate 96.4 %
Composition of Example 12: Compressive deformation rate 95.5 %

### Application Example 1: Cooling sheet

A heated solution obtained in the same manner except for adding 0.05% by weight of 1-menthol to the preparation of Example 2 and dissolving menthol into a butylenes glycol solution in which parabene was dissolved was filled in a blister mold having a concave part with the maximum size for each of length, width and depth of about 4.5 cm × 11 cm × 4 mm and molded to obtain a cooling sheet.

When the cooling sheet was applied to a forehead, it kept a state sticked to the forehead even if a head was moved. Further, while it was sticked to the forehead for about 3 hours, cooling feeling was kept for the forehead. Further, upon peeling the cooling sheet from the forehead, it could be peeled neatly without flexing or breakage by pulling.

When a support made of a non-woven fabric is previously installed along the inner wall of the blister mold, a cooling sheet having a support on one surface can be obtained by filling the heated solution into the blister mold.

This can reinforce the cooling sheet per se and when the cooling sheet is applied to a forehead or the like, the support can be used as a fixing band by extending the support in the longitudinal direction and turning the support around the head.

### Application Example 2: Cooling sheet

A heated solution was prepared in the same manner as described above except for further adding 0.05% by weight of 1-menthol to the same preparation as that of Example 7 and dissolving menthol into a butylene glycol solution to which parabene was dissolved.

A gel ingredient comprising κ carrageenan, 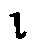 carrageenan, locust bean gum, glucomannan and a partially neutralized polyacrylic acid was added little by little while stirring thoroughly to about a half amount of butylene glycol to obtain a moistened liquid gel ingredient. Parabene and 1-menthol were added to the remaining butylene glycol and dissolved completely while stirring under heating to obtain a solution. After cooling, it was added little by little to the moistened liquid gel ingredient described above while stirring thoroughly. It was added to a predetermined amount of water while stirring thoroughly, to which sorbitol was added to swell the gel ingredient. The obtained swollen liquid gel ingredient was heated to about 85°C and when the gel ingredient under swelling was dissolved homogeneously, a separately prepared aqueous solution of crosslinking agent was added dropwise under stirring and maintained at 85°C for about 30 minutes. Then, it was immersed in a water bath controlled at 70 to 75 °c to lower the solution temperature and hold it till the bubbles in the solution were eliminated.

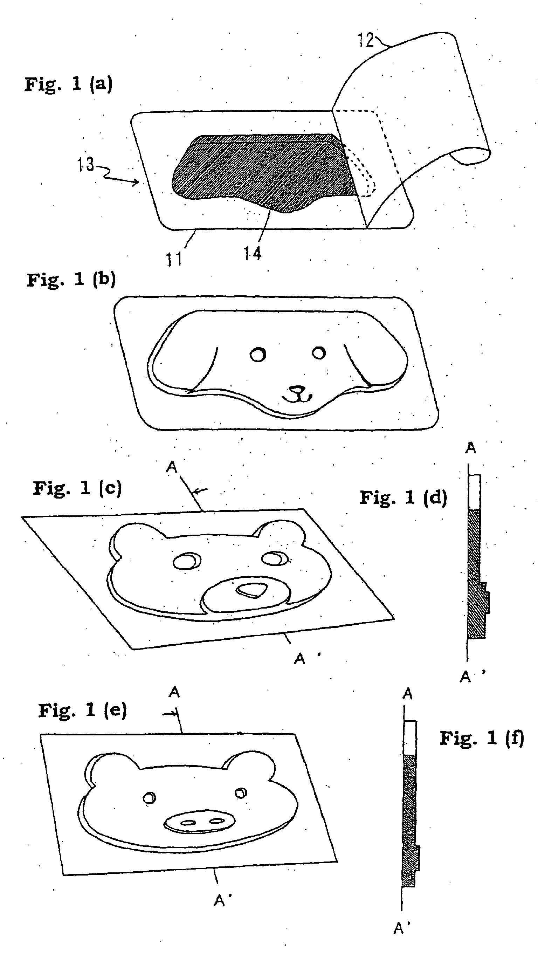

Thus obtained heated solution for forming the gel composition was filled in a blister vessel 11 as shown in Fig. 1(a), sealed with an aluminum lamination film 12 by easy sealing and a cooling sheet 14 of a predetermined shape contained in a portable type gel pack material 13 was molded.

Since the cooling sheet 14 has a shape conforming to the upper edge of an eyebrow at the applying portion, the weight of the cooling sheet can be supported at this portion. Accordingly, the sticking ability can be satisfied sufficiently not only during laying down but also in active state such as during deskwork or walking. Further, since it has no support such as non-woven fabric as in the existent cooling sheet and since the cooling sheet itself is transparent, it gives no unwholesome appearance and can be used with no sense of discomfort even in an office room.

Further, in a case of forming the concave bottom of the blister vessel, for example, as a face of a dog as shown in Fig. 1(b), a face of a bear as shown in Fig. 1(c) and (d), or a face of a pig as shown in Fig. 1(e) and (f) in a relief, since the configuration is transferred to the filled gel, it is preferred as a children's cooling sheet.

### Application Example 3: Face sheet

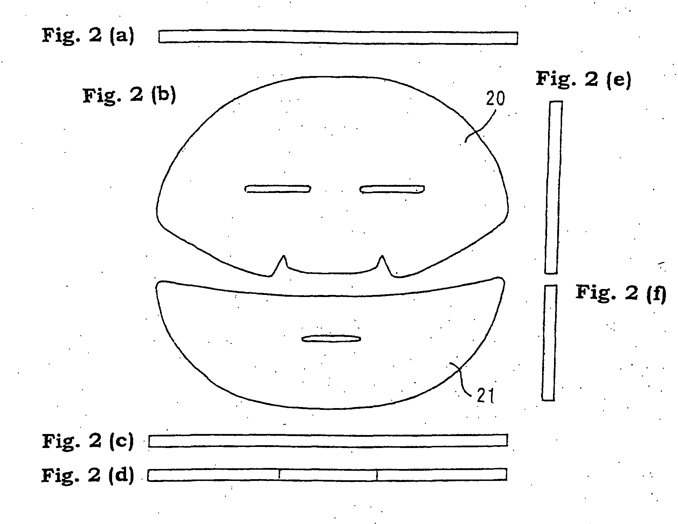

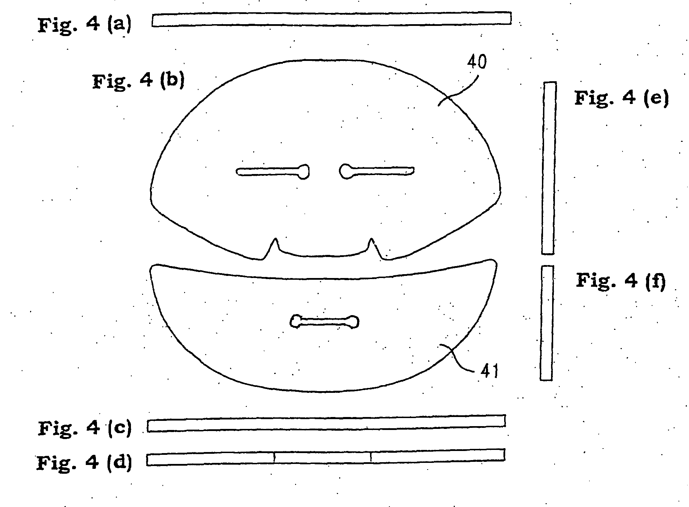

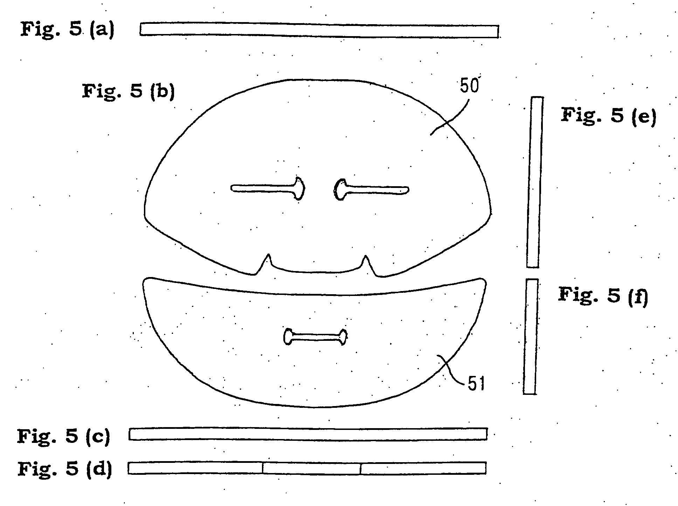

A heated solution of the same preparation as that of Example 2 was filled and molded in a blister mold with a thickness of about 2 mm and face sheets 20 and 21, 30 and 31, 40 and 41, and 50 and 51 having the shapes as shown in Fig. 2 to Fig. 5 were prepared, respectively.

When the face sheets were sticked over the entire face, they kept a state sticked to the face even when a head was moved. Further, when they were applied to the face, they favorably conform to the evenness of the face. Further, after sticking to the face for about one hour and peeling them, water retainability on the face was maintained. In addition, they could be peeled neatly by pulling upon peeling with no breakage.

Further, with the shape as shown in Fig. 2 to Fig. 5, the gel composition could completely cover the entire face in the form conforming to the shape of eyes or mouth based on the characteristics of the gel composition by extending or pulling the gel composition. Further, the shape of the gel compositions could be changed for the size and the position of the eyes and the mouth also depending on the individual difference to fit them for the entire face.

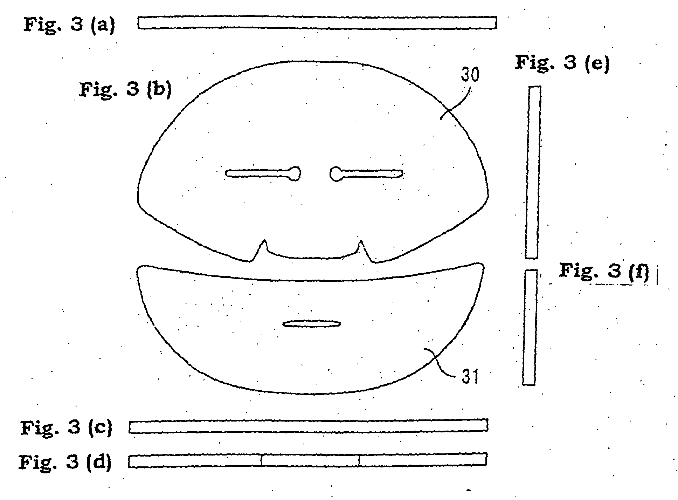

Further, as shown in Fig. 3 to Fig. 5, since the ends of a holes 23 corresponding to the eyes or the mouth are rounded at 24, breakage did not occur from the ends even when the face sheet was stretched and shrunk. Further, since it is possible to mold the face sheet somewhat smaller than the face and stretch the entire portion upon use into a size fitting the face, it can be provided as a compact size.

### Application Example 4: Eye pack

A heated solution of the same preparation as that of Example 2 was filled and molded in a crescent shape blister mold having a length of about 10 cm, a width of about 2 cm and a thickness of 2 mm to obtain an eye pack.

When the face sheet was applied to the vicinity of eyes, it was kept at a state sticked in the vicinity of eyes even when the head was moved. Further, after sticking for about 3 hours to the vicinity of the eyes and then peeling, water retainability for the vicinity of eye was kept.

### Application Example 5: Eye pack

Vegetable collagen, trehalose, α-hydroxy acid or trimethylglycine was added by a slight amount to the preparation of Example 4 to prepare a heated solution.

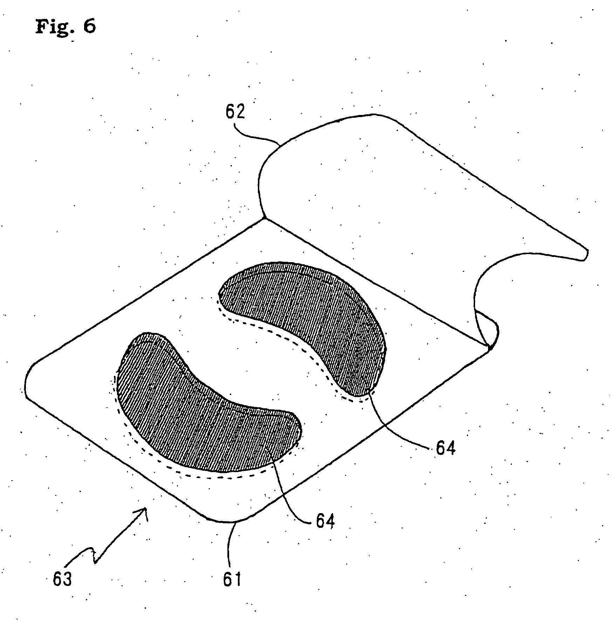

The obtained solution was filled in a blister pack vessel 61 having a pair of crescent-shape concave parts as shown in Fig. 6, sealed with an aluminum lamination film 62 by easy sealing, and molded into a pair of crescent shape eye packs 64 contained in a portable type gel pack 63.

Since the eye pack 64 has a high compressive deformation rate, when it is taken out of the blister pack vessel 61, it had a sufficient compressive strength against gripping pressure even when the thickness was thin (for example, about 2 mm), and possessed a sufficient strength also against a tensile force exerting upon sticking to the vicinity of eyes or adjusting the position and, they were free from tearing, breaking, or cracking and extremely easy to handle with.

Further, after sticking, it was extremely excellent in the feeling of fitness due to the resiliency of the gel sheet and had sufficient adhesiveness irrespective of small close contactness.

### Application Example 6: Lip pack

Vegetable collagen, trehalose, α-hydroxy acid or trimethylglycin and olive oil were added in a slight amount to the preparation of Example 6 to prepare a heated solution.

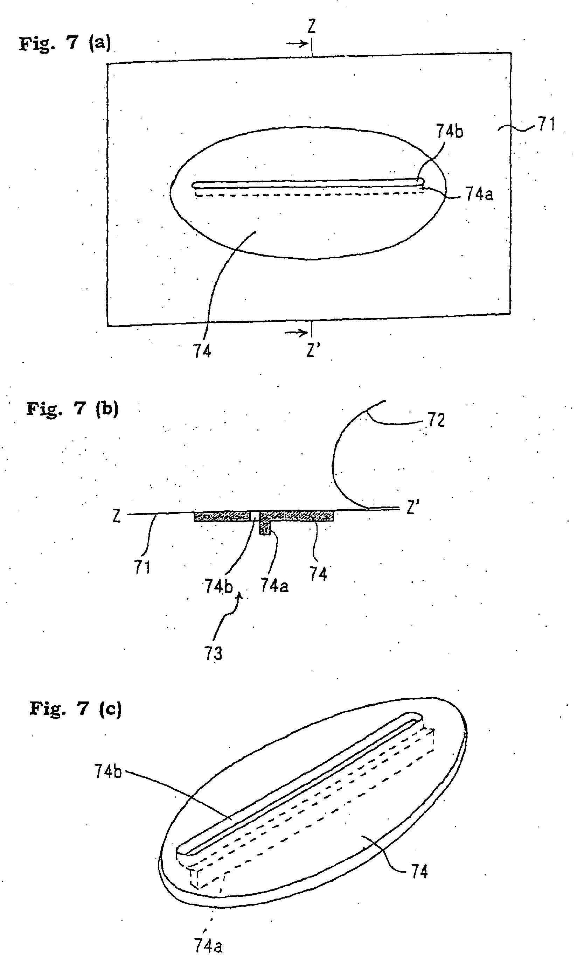

The obtained solution was filled in a blister pack vessel 71 having a concave shape as shown in Fig 7(a) to (c), sealed with an aluminum lamination film 72 by easy sealing and a lip pack 74 contained in a portable type gel pack material 73 was molded.

Since the lip pack 74 had a high compressive deformation rate, it was not plucked, torn or cracked even upon taking out of the blister pack vessel 71 of a complicate concave shape and extremely easy to handle with. Further, it can be extended by pulling by both hands, is not broken even when extended into an extremely thin thickness and gives feeling of fitting when sticked in the thinned state to a lip. Further, a ridge 74a of the lip pack 74 is sticked to upper surface of a lower lip thereby capable of avoiding displacing downward slide, taking care of the lower lip substantially entirely, providing sufficient moistening to a dried lip while preventing drying thereof, and preventing cracking or the like.

Further, conversation is not hindered even during sticking of the pack due to a long slit 74b perforated through the lip pack 74.

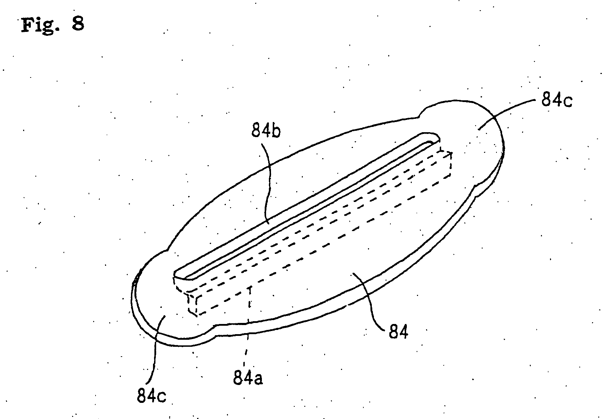

Further, as a modified example, as shown in Fig. 8, weakening of the strength caused by the long slit 84b can be compensated by forming a bulged part 84c to a portion corresponding to the end of the lip. Further, since the area of adhesion available is increased, it can be adhered snugly to the end of the lip.

### Application Example 7: Gel sheet for treatment of sunburn/glow

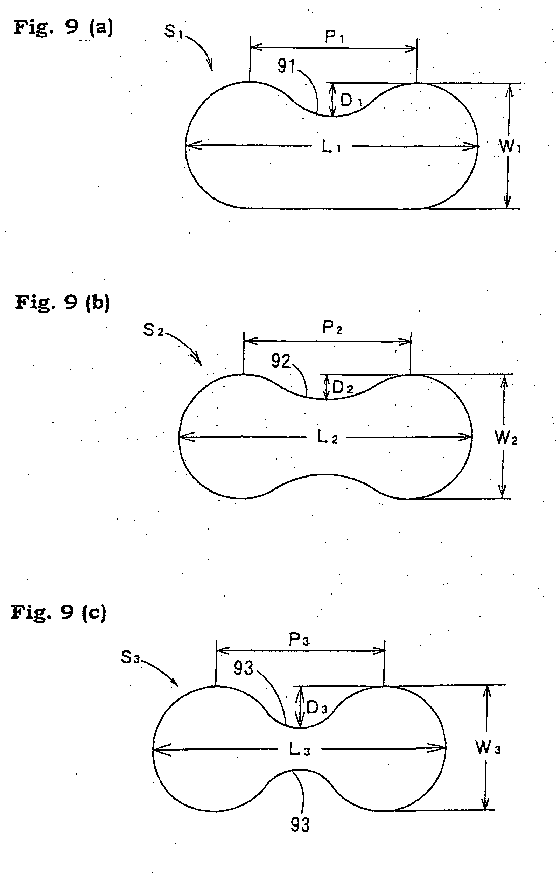

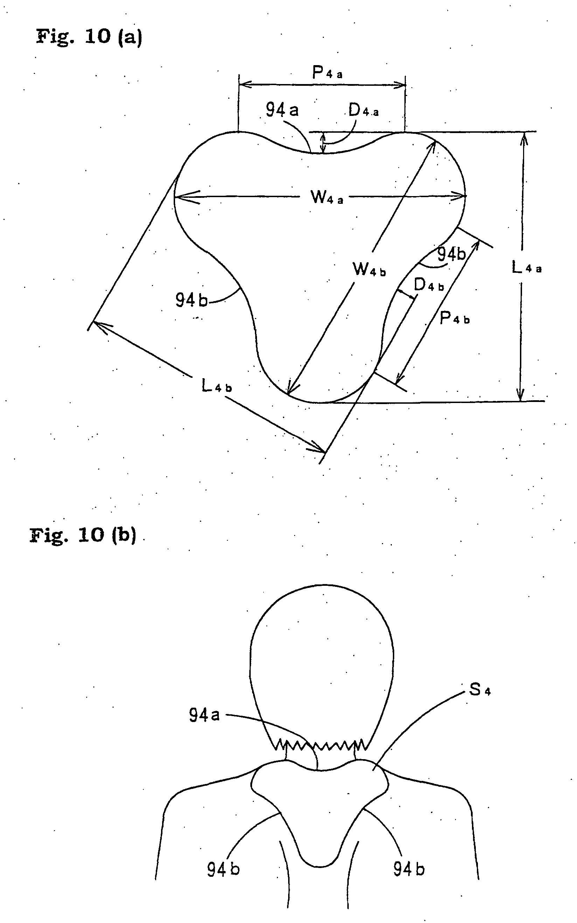

A heated solution of a preparation of Example 10 was prepared to mold gel sheets shown in Fig. 9(a) to (c) and Fig. 10(a).

In a gel sheet S₁ shown in Fig. 9(a), the sheet is entirely oval in which one concave recess 91 is formed along the minor axis of the sheet. The gel sheet S₁ is defined as 2 mm thickness, 200 mm length L₁ for the longest portion in the longitudinal direction, 90 mm length W₁ for the longest portion in view of the width, 25 mm depth D₁ for the concave recess and 110 mm width P₁ for the concave recess.

In a gel sheet S₂ in Fig. 9(b), the sheet is entirely oval or elliptic, which is substantially identical with the gel sheet S₁ excepting that the concave recesses 92, 92 are formed by two opposing in view of the minor axis of the sheet.

A gel sheet S₃ in Fig. 9(c) is substantially identical with the gel sheet S₂ excepting that the depth D₃ is 30 mm for each of the concave recesses 93, 93.

In a gel sheet S₄ in Fig. 10(a), the entire sheet is substantially trigonal (substantially isosceles triangle), in which concave recesses 94a, 94b and 94b are formed respectively to each side of the sheet. The gel sheet S₄ is defined as 2 mm thickness, 150 mm length L₄ for the longest portion in the longitudinal (height) direction and 120 mm length W₄ for the longest portion for the width. Further, the concave recess 94a is defined as 10 mm depth D₄ₐ, 70 mm width P₄ₐ and each concave recess 94b is defined as 10 mm depth D_{4b}, and 65 mm width P_{4b}.

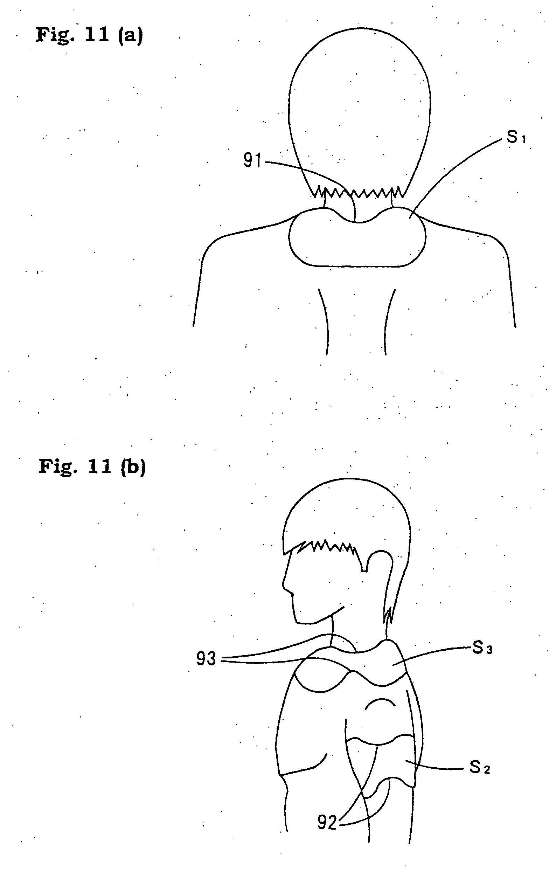

When the gel sheet is applied to skins suffering from smarting or itching feeling due to sunburn caused by sea bathing, etc. such symptoms can be mitigated. For example, as shown in Fig. 11(a), in a case of sticking the gel sheet S₁ near the neck line on the back of a body, when it is sticked while conforming the edge of the concave recess 91 with the neck line (base of neck), close adhesion of the gel sheet S₁ to the curved surface of a human body is enhanced and it less peels even upon movement of the body. That is, when an existent rectangular gel sheet is applied near the neck line on the back, while it is sometimes adhered to some extent to the skin due to the soft and flexibility of the gel body, it tends to peel off at the edge near the neck line of a large curvature, and the peeling prevails from a peeled portion of the edge to the entire sheet. On the contrary, in the gel sheet S₁ according to the invention, since the concave recess 91 exerts so as to conform the curved surface of the body, the periphery is adhered easily over the entire circumference to the skin and does not cause peeling from the partial relief to the entire sheet surface as in the existent case. In the gel sheet Si having only one concave recess 91, since the portion of the back below the neckline of the human body is relatively flat, the linear edge corresponding to the concave recess 91 may be sticked to the portion.

Further, as shown in Fig. 11(b), a gel sheet S₂ is sticked while directing each of the concave recesses 92, 92 in the longitudinal direction of an arm, and a gel sheet S₃ is sticked while conforming the edge of one concave recess 93 along the base of a neck and riding the other concave recess 93 over the shoulder (edge). Since the gel sheet S₂, S₃ each has a pair of opposing concave recesses at the peripheral portion respectively, they have higher adhesion to the curved surface of the human body. Particularly, the adhesion at the edge of the curved portion of the sheet sticked to the arm or the shoulder is improved compared with the gel sheet Si, and it is suitable for applying to the portion having the large curvature such as an arm or a shoulder.

Further, as shown in Fig. 10(b), a gel sheet S₄ can be sticked while conforming the edge of the concave recess 94a along the neck line and the edges of the concave recesses 94b, 94b along the hem of the raised portion of right and left bladebones, and this is suitable for applying to the portion of the human body.

In the gel sheet S₂ and the gel sheet S₃, the size may be different between one concave recess and the other concave recess. Further, in the gel sheet S₄, the concave recess 94a may be saved leaving the edge linear, or one or both of the concave recesses 94b and 94b may be omitted.

Since the gel sheet for treating sunburn/glowing has high compressive deformation rate, it extends effectively, can increase the area of adhesion by reducing the thickness and can be sticked over a wide range. Further, since the concave recess functions so as to conform to the curved surface of the body, the edge for the curved portion of the gel sheet sticked to the curved surface of the human skin is sticked snugly without raising from the skin, the peripheral portion is easily adhered over the entire circumference to the skins and is less peeled even movement of the body.

### Application Example 8: Gel patch for insect sting

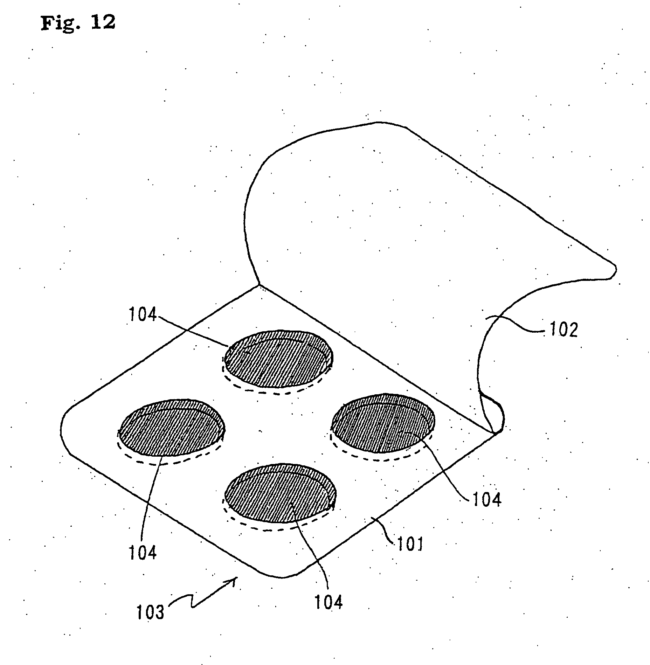

A heated solution prepared by adding 0.03% by weight of 1-menthol and 1% by weight of diphenhydramine hydrochloride to a preparation of Example 11 was filled in a blister pack vessel 101 of a disk-like shape (about 2 cm diameter, about 2 mm thickness) having plural concave parts shown in Fig. 12 and tightly sealed by an aluminum lamination film 102 by easy sealing to mold pack 104 for insect sting contained in a portable gel pack material 103 for insect sting.

Since the patch 104 for insect sting retains a sufficient amount of water content compared with the existent patch, it exhibits an excellent effect for preventing itching also with addition of cooling effect against inflammation.

### Application Example 9: Patch for pimple

A heated solution formed by adding 0.3% by weight of isopropylmethyl phenyl to a preparation of Example 12 was filled in a similar vessel to the disk-like blister pack vessel having several concave parts shown in Fig. 12 and tightly sealed to mold a pack for pimple.

Since the patch for pimple is substantially transparent, it is not conspicuous when used for face or the like, and since it retains a sufficient amount of water content, it exhibits an excellent effect for preventing itchy feeling in addition to the cooling effect for inflammation.

### Application Example 10: Gel cake substrate

A heated solution of the preparation of Example 6 was prepared, and a soft and flexible resin foam comprising open cells such as sponge was immersed in the solution to sufficiently absorb the solution into the open cells and then it was taken out to obtain a block-form gel cake substrate.

In the gel cake substrate, since a gel composition having a high compressive deformation rate is integrated with the soft and flexible resin form and since the gel composition effectively conforms the shrinkage of the resin foam, the gel composition is not crumbled from the resin foam.

Further, the block-form gel cake substrate can be used by property cutting into a sheet-like shape or a specified shape depending on the application use.

Further, for releasing water from the gel composition carried on the gel cake substrate, when an effective ingredient such as urea is blended or a potassium glycerin solution is used in place of water upon preparation to the gel composition, release of water from the gel composition is promoted upon shrinking under squeezing of the gel cake substrate and the effective ingredient can be discharged to prepare a gel cake effective for skin roughing.

A wound pad can be prepared by blending a sterilizing or bacteria static agent or effective ingredients to wounds.

Further, pad for bedsores can be prepared by blending a relatively great amount of succharide and an effective ingredient to wounds, for example, SHIUNKO, povidone-iodine.

### Application Example 11: Cooling sheet

0.03% by weight of 1-menthol was further added to the same preparation as Example 7 to prepare a heated solution, and a cooling sheet was formed in the same manner as in Application Example 2.

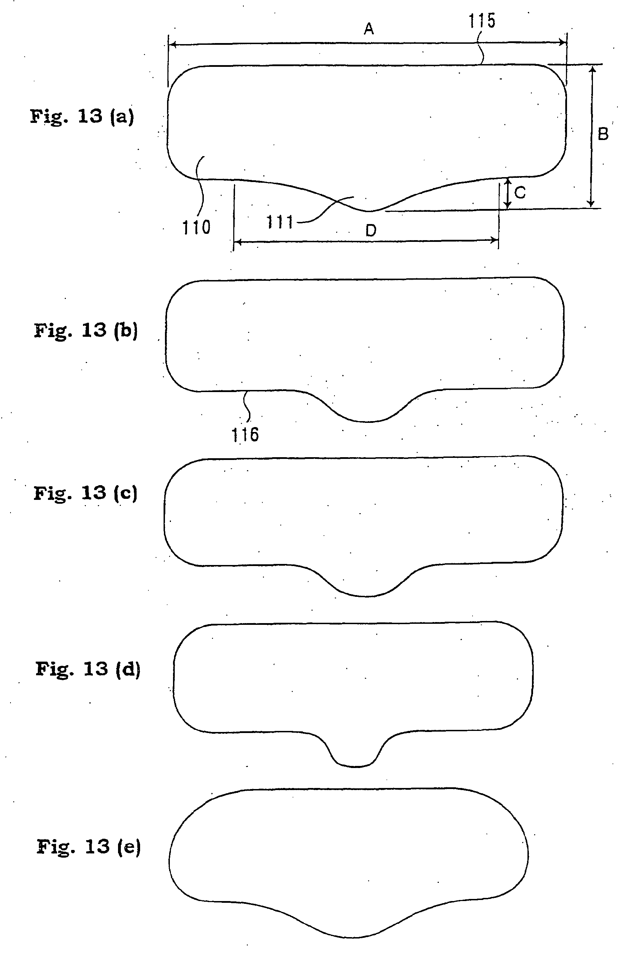

As shown in Fig. 13(a) to (e), a cooling sheet 110 has an extension 111 each at the center of a lower edge 116. The longitudinal length B for the longest portion is 45 mm and the lateral length A for the longest portion is 110 mm and the thickness is 3 mm. The height C of the extension 111 is 10 mm and the width D of the extension is 80 mm, 35 mm, 40 mm, 30 mm and 80 mm, respectively, for each of the curves.

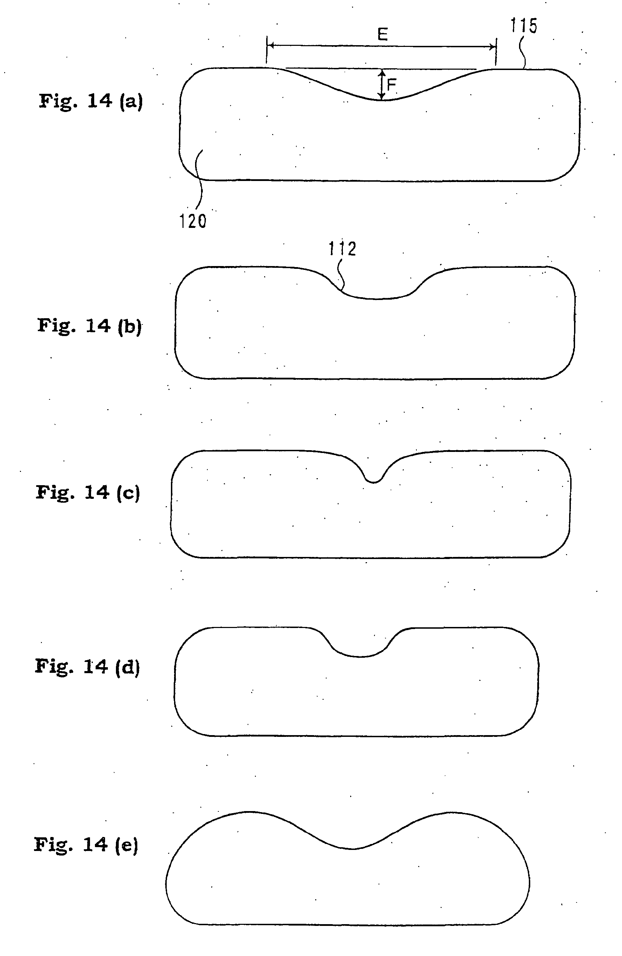

Further, as shown in Fig. 14(a) to (e), the cooling sheet 120 has a concave recess 112 each at the center of an upper edge 115. The longitudinal length B at the longest portion is 45 mm and the lateral length A at the longest portion is 120 mm and the thickness is 3 mm. The depth F of the concave recess 112 is 10 mm and the width E at the concave recess is 80 mm, 35 mm, 40 mm, 30 mm, and 80 mm for each of the curves.

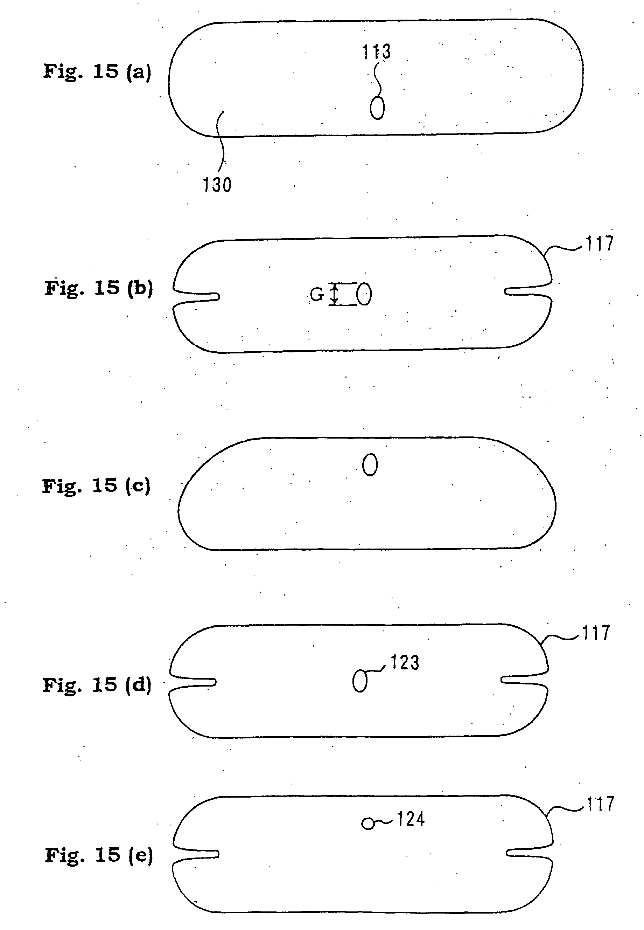

As shown in Fig. 15(a) to (e), another cooling sheet 130 has an elliptic convex part 113, concave part 123 or circular through hole 124 formed at an upper portion or at a center on the longitudinal center line of the surface of the cooling sheet 130 instead of the concave recess or the extension 112, 111 at the upper or lower edge 115, 116 shown in Fig. 12 and Fig. 13. The height of the convex part 113 and the concave part 123 is about 3 mm, the diameter G of the convex part is about 10 mm and the diameter of the through hole 24 is about 5 mm.

### Application Example 12: Cooling sheet

0.03% by weight of 1-menthol was further added to the same preparation as Example 7 to prepare a heated solution and a cooling sheet was formed in the same manner as in Application Example 2.

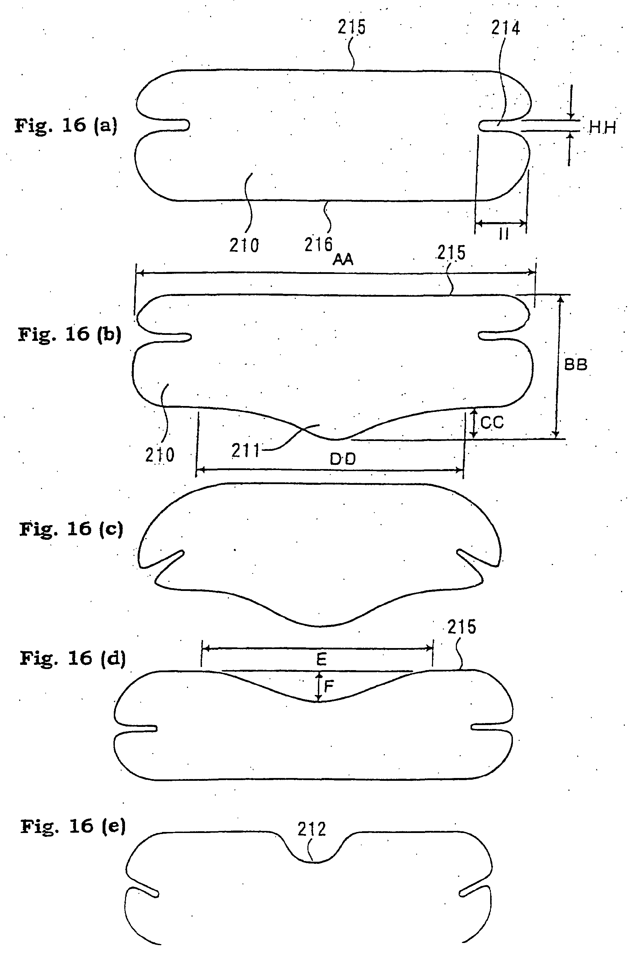

As shown in Fig. 16(a) and (b), a cooling sheet 210 has a longitudinal length BB of 45 mm at the longest portion and the lateral length AA of 110 mm at the longest portion and a thickness of 3 mm. The width HH of a notch 214 is 2 mm, the length II of the notch is 20 mm and the notch 214 is formed around the center perpendicularly to both of side edges 217.

In Fig. 16(b), a notch 214 is formed like in Fig. 16(a) perpendicularly to both sides 217 at about 1/3 position from upper edge, while an extension 211 is formed at the lower edge 216. In Fig. 16(c), it is formed being inclined at an angle of about 45° on both side edges 217 at about 2/3 position from upper edge, and an extension 211 is formed in the same manner as in Fig. 16(b). In Fig. 16(d), it is formed perpendicularly to both side edges 217 at about 2/3 position from upper edge, and a concave recess 212 is formed at an upper edge 215. In Fig. 16(e), it is formed being inclined at an angle of about 45° at the central position between both edges 217 and a concave recess 212 is formed like in Fig. 16(d).

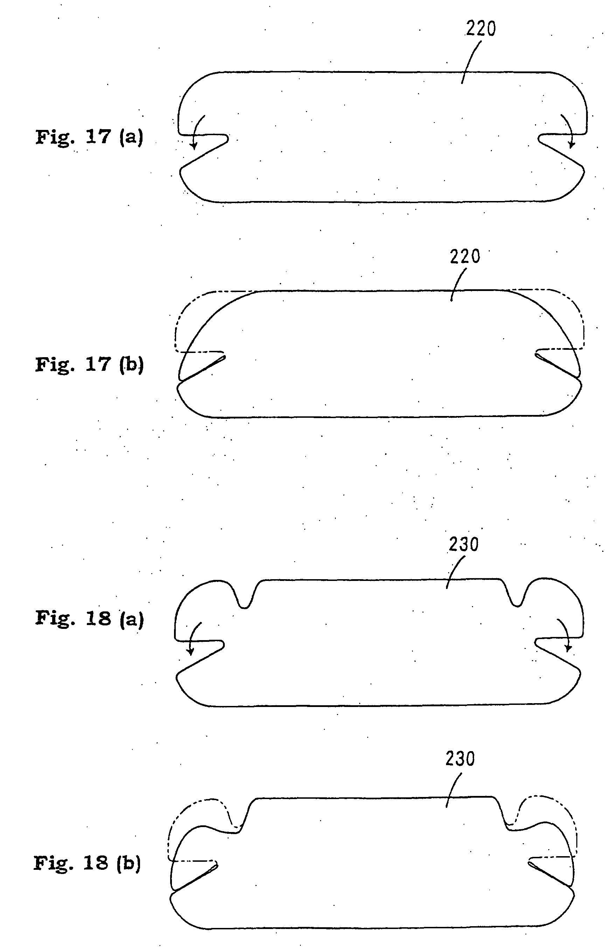

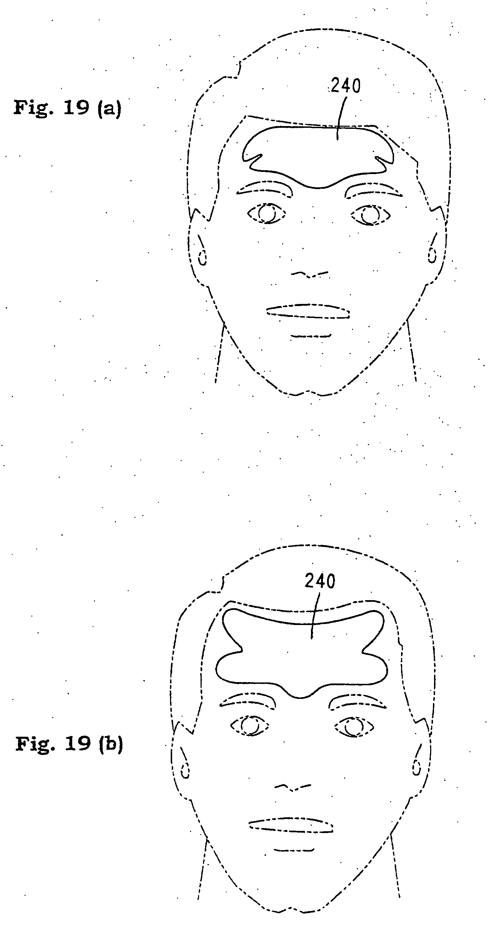

For example, in a case of using the cooling sheet 220 or 230 shown in Fig. 17(a) and Fig. 18(a), the cooling sheets 220 and 230 are used with the upper end thereof being bent downward so as to close the notch as shown in Fig.17(a) and Fig.18(a). Specifically, as shown in Fig. 19(a), in a case where the forehead is narrow, the cooling sheet 240 can be sticked so as not to cover as far as the boarder of hair with the cooling sheet by bending the upper end thereof downward so as to close the notch. Further, as shown in Fig. 19(b), in a case of receded hair, the cooling sheet 240 can be sticked so as to reach the upper portion of the forehead by stretching a portion above the notch.

### Application Example 13: Cooling sheet

0.03% by weight of 1-menthol was further added to the same preparation as Example 7 to prepare a heated solution, and a cooling sheet was formed in the same manner as in Application Example 2.

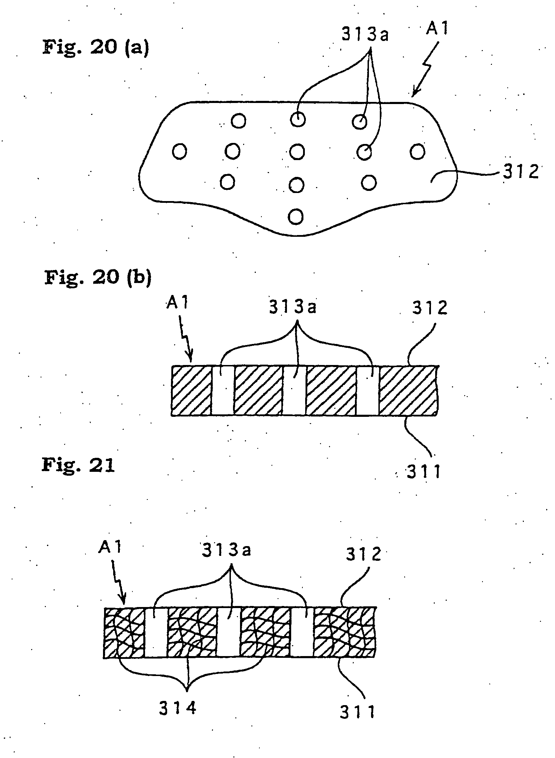

As shown in Fig. 20, in a cooling sheet A1, plural circular through holes 313a extending from an sticked surface 311 to an opposite surface 312 are formed at appropriate positions of the sticked surface 311 to be sticked to the forehead as a heat dissipation promoting portion for dissipating heat by increasing the area of contact with external air.

Thus, water content in the pad material A1 is effectively evaporated through the through holes 313a to dissipate heat by the heat conducting from a human body to the pad material A1 thereby preventing warming of the pad material A1 and providing a sufficient cold insulation effect for a long time.

Further, for easy movement of liquid inside the pad material A1 and efficient evaporation of the water content in the pad material A1 through the through holes 313a, etc. to conduct heat dissipation, fibers 314 having hydrophilic property at least on the surface are dispersed inside the pad material A 1 as shown in Fig. 21.

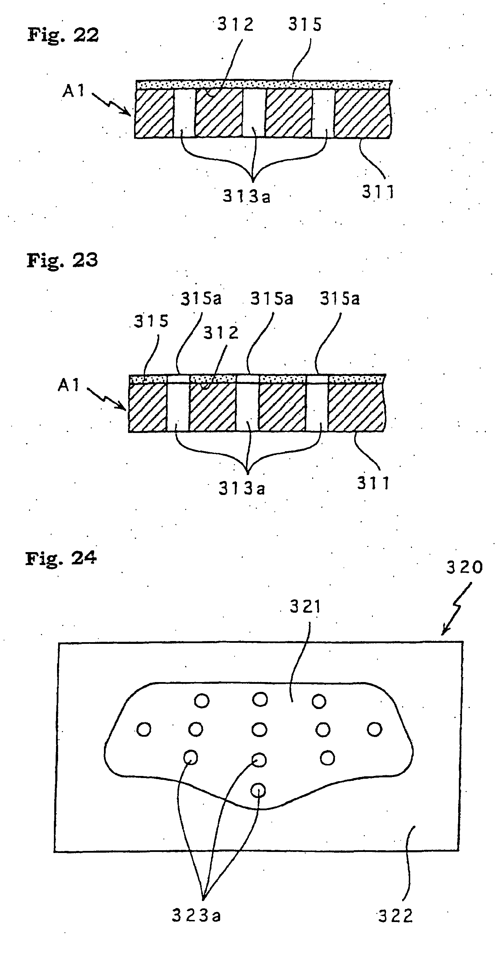

Further, in order to prevent deposition of dusts or dirts to the surface 312 opposite to the sticked surface 311 of the pad material A1, or deposition of the surface 312 on the opposite side to other portion thereby defoliating the pad material A1 from the sticking portion, as shown in Fig. 22, an air permeable sheet 315 such as made of non-woven fabric is disposed for the entire surface 312 opposite to the sticked surface 311 as shown in Fig. 22. As shown in Fig. 23, apertures 315a may also be formed to the air permeable sheet 315 corresponding to the through holes 313a such that evaporation of the water content through holes 313a is not suppressed by the air permeable sheet 315.

Further, when a portable pad material making the pad material A1 portable is prepared, a vessel 320 comprising a containment concave part 321 having a planar shape corresponding to the planar shape of the pad material A1 and a flange 322 extended at the periphery for the opening of the containment concave part 321 is used as shown in Fig. 24. In the vessel 320, plural convex parts 323a reaching the opening at the upper surface of the containment concave part 321 are formed so as to correspond to the through holes 313a in the pad material A1.

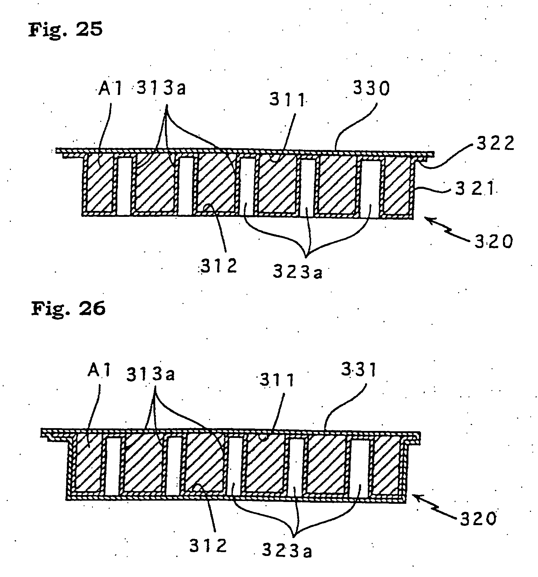

Then, as shown in Fig. 25, a gel composition is filled in the containment concave part 321 of the vessel 320, and the gel composition is gelled to form a pad material A1 having plural through holes 313a which are formed corresponding to the convex part 323a in the containment concave part 321, and then an airtight film 330 is covered so as to close the opening of the containment concave part 321 of the vessel 320, and finally the airtight film 330 is releasably adhered to the flange 322 of the vessel 320 to seal the containment concave part 321 of the vessel 320 in a state containing the pad material A1.

This suppresses evaporation of the water content from the pad material A1 by sealing with the airtight film 330 and it can be stored for long time, distributed and carried about in this state.

Further, in this constitution, since the pad material A1 engages the plural convex parts 323a, rattling of the pad material A1 in the vessel can be prevented and it can be distributed and carried about in a state of upstanding the vessel.

Then, in a case of using the pad material A1 sealed by the airtight film 330 in the containment concave part 321 of the vessel 320, the airtight film 330 is pulled and peeled from the flange 322 of the vessel 320 thereby opening the containment concave part 321 of the vessel 310, then pad material A1 is taken out from the inside of the containment concave part 321 of the vessel 320, and the sticked surface 311 of the pad material A1 is sticked to the forehead at the sticking portion.

Further, as shown in Fig. 26. the vessel 320 containing the pad material A1 in the containment concave part 321 can be contained and sealed in a bag 331.

### Application Example 14: Cooling sheet

0.03% by weight of 1-menthol was further added to the same preparation as Example 7 to prepare a heated solution, and a cooling sheet was formed in the same manner as in Application Example 2.

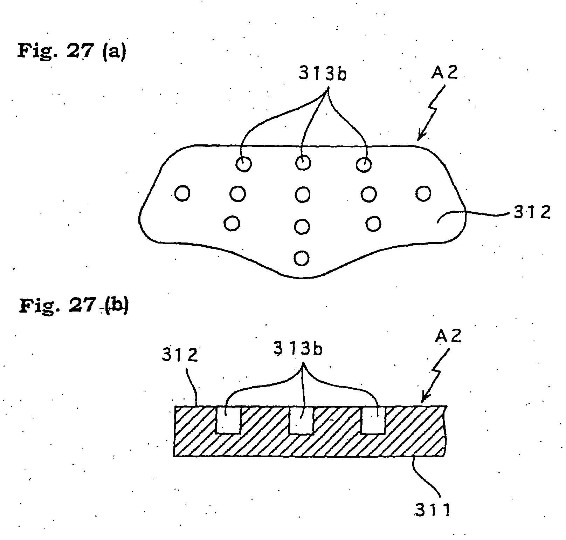

In the pad material A2, as shown in Fig. 27(a) and (b), plural heat dissipating concave parts 313b are formed on the surface 312 opposite to the sticked surface 311 as the heat dissipation promoting portion.

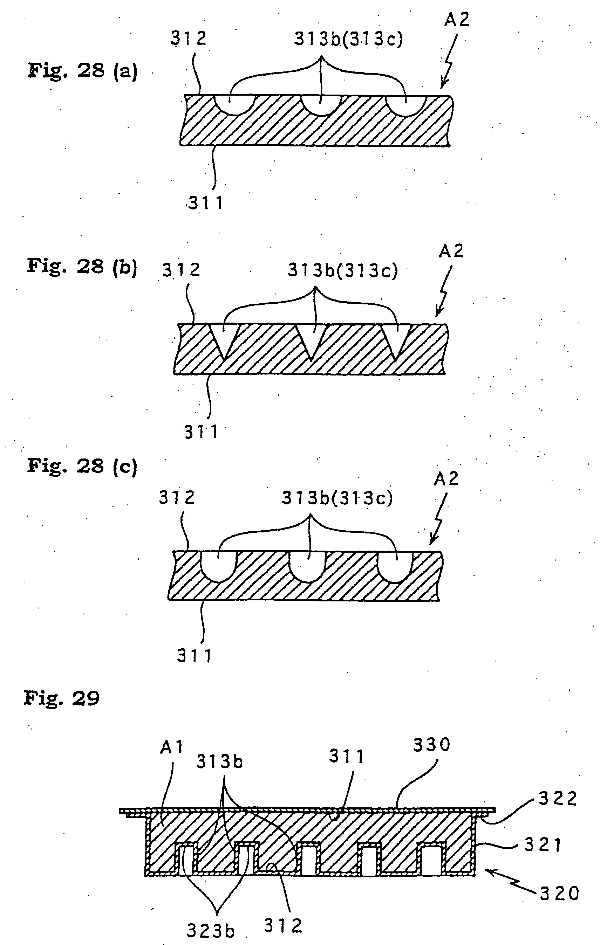

The concave parts 313b may have a cross sectional shape, for example, as shown in Fig. 28(a) to (c).

For manufacturing a portable pad material for enabling the pad material A2 to be carried about, as shown in Fig. 29, a gel composition is filled in the containment concave part 321 of a vessel 320, the gel composition is gelled to form a pad material A2 having plural heat dissipating concave parts 313b formed corresponding to convex parts 323b in the containment concave part 321, then an airtight film 330 is covered so as to close the opening of the containment concave part 321 of the vessel 320, the airtight film 330 is releasably adhered to a flange 322 of the vessel 320, and the containment concave part 21 of the vessel 320 is sealed in a state of containing the pad material A2.

### Application Example 15: Cooling sheet

0.03% by weight of 1-menthol was further added to the same preparation as Example 7 to prepare a heated solution, and a cooling sheet was formed in the same manner as in Application Example 2.

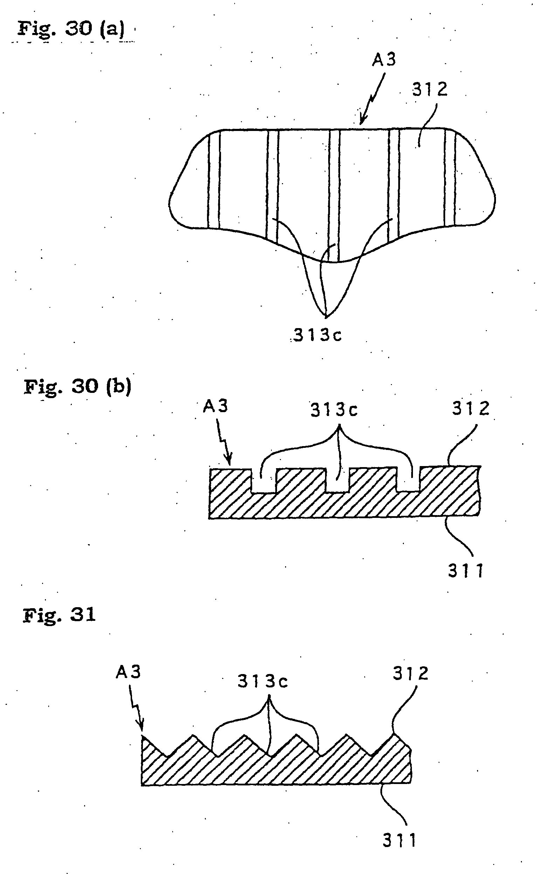

As shown in Fig. 30, in a pad material A3, plural heat dissipating grooves 313c in the form of trenches are formed in a surface 312 opposite to an sticked surface 311 as a heat dissipation promoting portion.

The cross sectional shape of the heat dissipating grooves 313c may be of a shape as shown in Fig. 28(a) to (c) or, heat dissipating grooves 313c may be disposed continuously in the surface 312 opposite to the sticked surface 311 as shown in Fig. 31.

### Application Example 16: Cooling sheet

0.03% by weight of 1-menthol was further added to the same preparation as Example 7 to prepare a heated solution, and a cooling sheet was formed in the same manner as in Application Example 2.

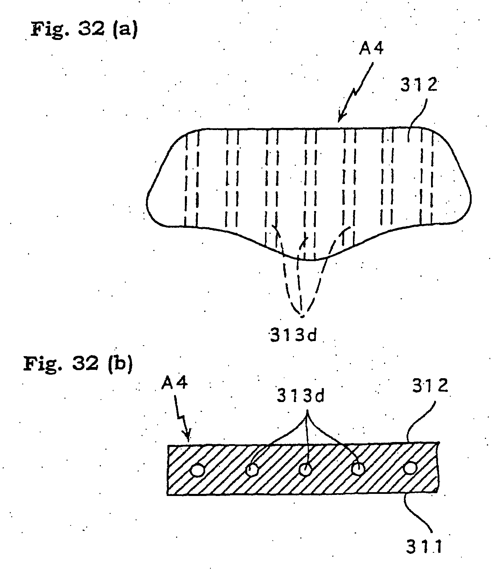

As shown in Fig. 32, in a pad material A4, plural heat dissipating channels 313d having a circular shape are disposed at appropriate positions in the thickness of the pad material A4 between an sticked surface 311 and an opposite surface 312 as the heat dissipation promoting portion.

### Application Example 17: Cooling sheet

0.03% by weight of 1-menthol was further added to the same preparation as Example 7 to prepare a heated solution, and a cooling sheet was formed in the same manner as in Application Example 2.

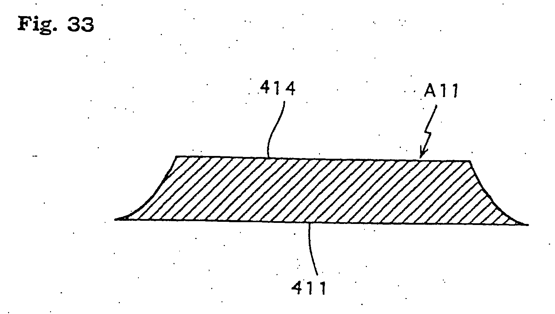

As shown in Fig. 33, a pad material A11 is formed as a substantially rectangular shape in the planar view, in which one surface is an sticked surface 411 to be sticked to an appropriate applying portion of a human body and it is formed in a reduced thickness such that the thickness for the entire periphery is concaved toward the side edge of the sticked surface 411.

Thus, the pad material A11 is sticked smoothly along the applying portion of the human body to increase the ratio of area of the sticked surface 411 relative to the amount of the gel composition and the pad material A11 can be sticked stably to the applying portion even in a case of increasing the amount of the gel composition or in a case where the adhesiveness at the sticked surface 411 is low.

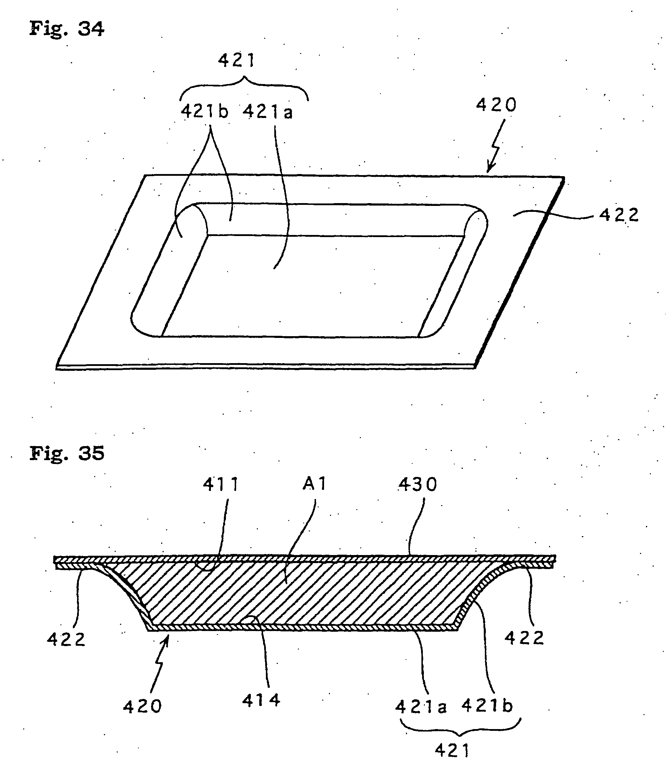

In manufacturing a portable pad material for enabling the pad material A11 to be carried about, as shown in Fig. 34, a vessel 420 comprising a containment concave part 421 for containing the pad material A11 and a flange 422 extended to the periphery of the opening of the containment concave part 421 is used.

In the vessel 420, the containment concave part 421 is formed to such a shape that the sticked surface 411 of the pad material A11 situates on the side of the opening of the containment concave part 421 and a peripheral wall 421 b of the containment concave part 421 is formed so as to diverge convexly from the end edge at the bottom 421a to the opening of the containment concave part 421.

Then, as shown in Fig. 35, a gel composition is filled into the containment concave part 421 of the vessel 420, and gelled to form a pad material A11 in the containment concave part 421, then an airtight film 430 is covered so as to close the opening of the containment concave part 421 of the vessel 420, the airtight film 430 is releasably adhered to the flange 422 of the vessel 420, and the containment concave part 421 of the vessel 420 is sealed in a state of containing the pad material A11.

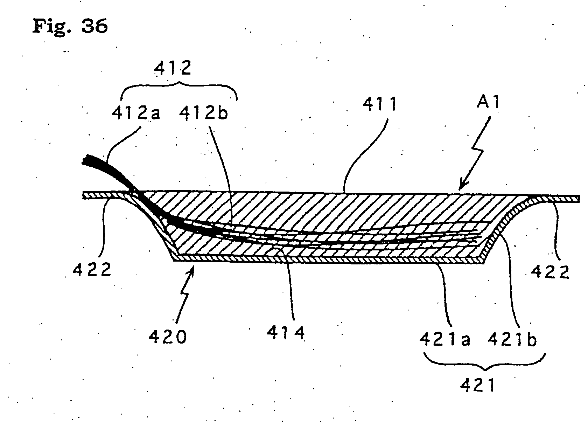

Further, in order that a finger or the like is kept from contact with the sticked surface 411 of the pad material A11 when the pad material A11 is taken out from the containment concave part 421 of the vessel 420, as shown in Fig. 36, a pad material take out member 412 having a portion protruding as a header part 412a out of the pad material A11 and a remaining part 412b engaging the pad material A11 may be provided. The pad material A11 may be taken out of the containment concave part 421 of the vessel 420 by picking the header part 412a of the pad material take-out member 412.

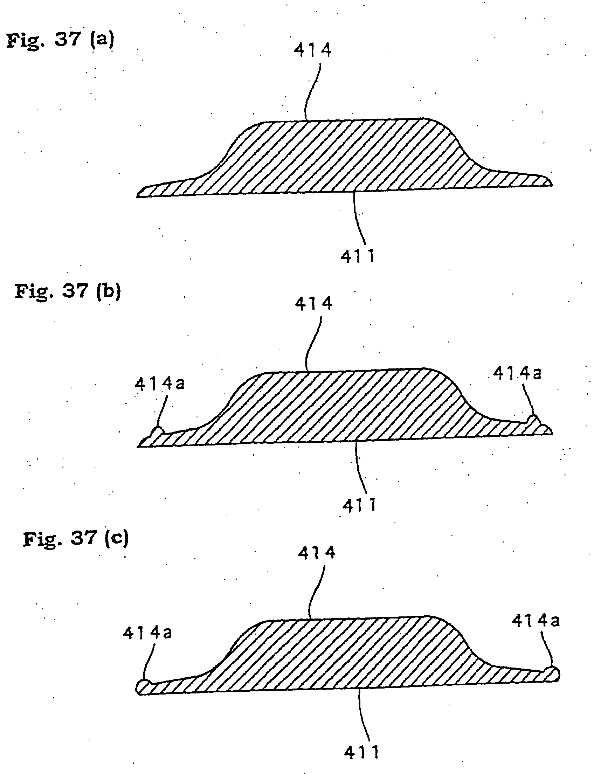

Further, as shown in Fig. 37(a), in a pad material A11, when a thin portion inclined moderately at the periphery of an sticked surface 411 is formed, the portion tends to conform to any concave/convex configuration at the applying portion to enable stable sticking to the applying portion. Further, when the thin portion with moderate inclination is formed, since the portion tends to be fractured, a reinforcing rib 414a is preferably formed on the surface 414 opposite to the sticked surface 411 at a position in the midway of a thin portion with moderate inclination, or at the end edge of the thin portion with moderate inclination.

### Application Example 18: Cooling sheet

0.03% by weight of 1-menthol was further added to the same preparation as Example 7 to prepare a heated solution and a cooling sheet was formed in the same manner as in Application Example 2.

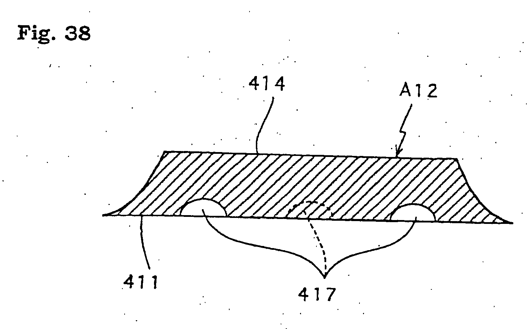

As shown in Fig. 38, in a pad material A12, plural sucking concave parts 417 are formed at appropriate positions on the sticked surface 411.

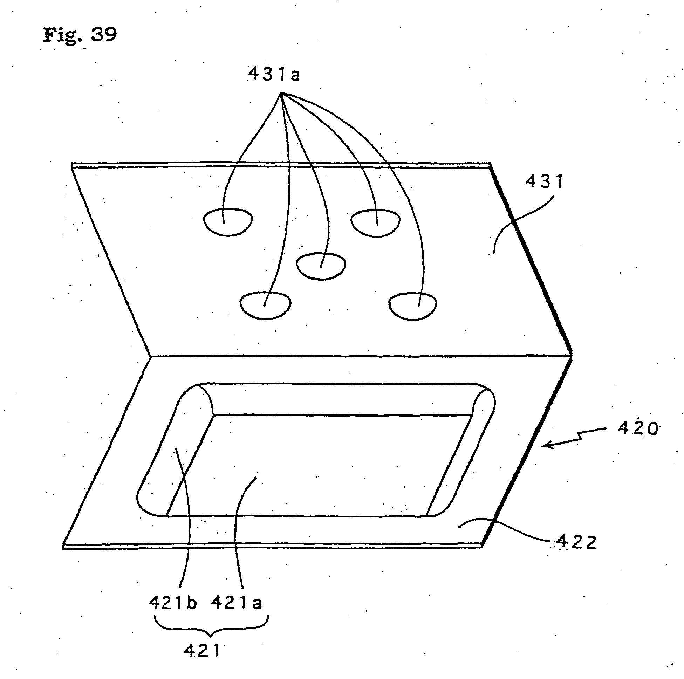

When a portable pad material for enabling the pad material A 12 to be carried about is manufactured, as shown in Fig. 39, a vessel 420 comprising a containment concave part 421 for containing a pad material A12 and a flange 422 extending at the periphery of the opening of the containment concave part 421 provided with a lid member 431 having convex parts 431a protruding on the side of the containment concave part 421 for closing the opening of the containment concave part 421 is used.

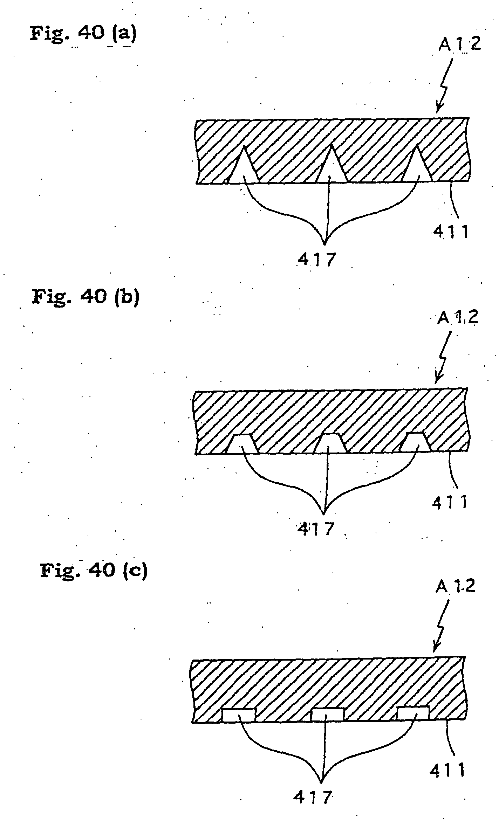

Further, the number, the shape, etc. of the sucking concave parts 417 formed on the sticked surface 411 of the pad material A12 have no particular restriction, and sucking concave parts 417 each of a conical shape, frusto conical shape or grooved shape may be disposed, for example, as shown in Fig. 40(a) to (c).

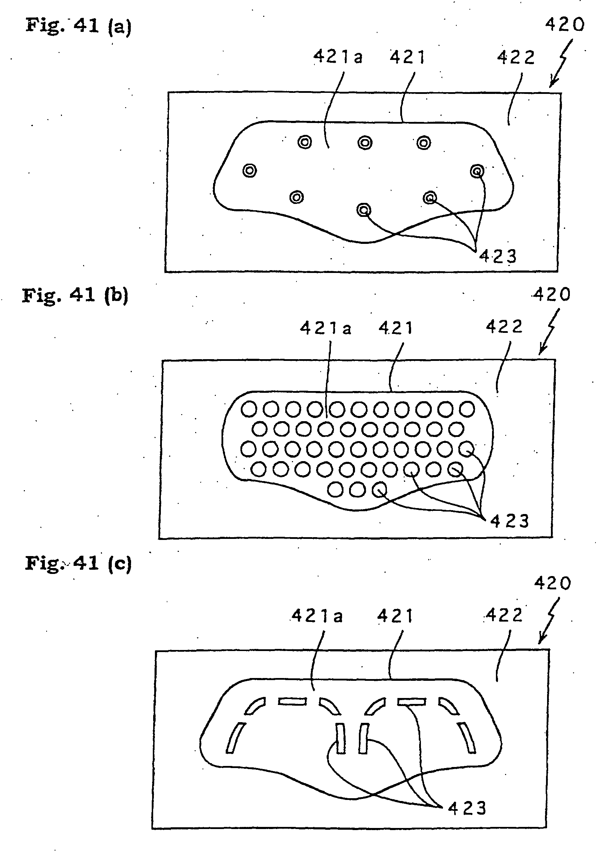

Further, as shown in Fig. 41 (a) to (c), it may be adapted such that the planar shape of a containment concave part 421 in a vessel 420 is formed into a planar shape corresponding to a forehead, and convex parts 423 each of a frusto conical shape are distributed dot-wise, convex parts 423 each of a circular shape are arranged in plurality, or convex parts 423 each of the short strand are arranged so as to form two hill shapes at a predetermined distance at a bottom 421a of the containment concave part 421.

According to the present invention, since the gel composition is formed of the water soluble polymer, has self-shape retainability, 30 to 95% by weight of the water content and the 65 to 99% of the compressive deformation rate, it is unexpectedly possible to prevent breaking, cracking and tearing during the handling of the gel composition and improve the strength and the handlability remarkably. In addition, since the gel composition per se can exhibit self-shape retainability with no support such as of a non-woven fabric, the adaptability can be enhanced more irrespective of the undulations, difference of curvatures and absence or presence of creases on the skin surface of a human body.

Particularly, in a case where the water soluble polymer constituting the gel composition is transparent, aesthetic appearance can be possessed or provided irrespective of the mode of use. Further, in a case where the water soluble polymer is edible, it can be used safely irrespective of the portion of use.

Further, in a case where the positioning part is formed to the pad material, it can be sticked being positioned to a forehead or the like easily without using a mirror or the like.

Further, in a case where notches are formed on both side edges, the material can be used being stretched or shrunk so as to conform the size of a forehead or a face accompanied by individual difference and it can be slicked appropriately to an appropriate portion.

Further, in a case of providing a heat dissipation promoting portion for dissipating heat by increasing the area of contact with external air, when heat is conducted from the human body or the like to the pad material, the water content and heat in the pad material can be evaporated and dissipated efficiently through the heat dissipation promoting portion thereby enabling to obtain sufficient cold insulation effect for a long time.

Furthermore, in a case where the sticked surface has sucking concave pads, when the pad material is urged to the applying portion of the human body, inside of the sucking concave part is evacuated, the pad material is sucked to the sticking surface of the human body and it can be sticked stably while preventing peeling.

## Claims

1. A topical gel composition formed of water soluble polymer having self-shape retainability, 30 to 95% by weight of water content, and 65 to 99% of a compressive deformation rate expressed by the maximum deformation rate till a partial fracture of the gel structure when the gel composition is compressed at a predetermined speed by using a plunger capable of pressing the gel composition at a constant speed.

2. The composition according to claim 1 which is transparent.

3. A pad material in which a topical gel composition formed of water soluble polymer having self-shape retainability, 30 to 95% by weight of water content, and 65 to 99% of a compressive deformation rate expressed by the maximum deformation rate till a partial fracture of the gel structure when the gel composition is compressed at a predetermined speed by using a plunger capable of pressing the gel composition at a constant speed has a sheet-like and rectangular or substantially rectangular shape, and has a positioning part formed at the center thereof.

4. The pad material according to claim 3, wherein the positioning part is a concave recess or an extension formed at the center of an upper edge or a lower edge, or a concave part, a convex part or a through hole formed on a vertical center line at the surface or rear face.

5. The pad material according to claim 4, wherein the concave recess or the extension has a width of 10 to 80 mm and a depth or height of 5 to 20 mm.

6. The pad material according to claim 4, wherein the height of the concave part or the convex part is 1 to 3 mm and the size of the concave part, the convex part or the through hole is 1 to 200 mm².

7. The pad material according to claim 3, wherein the pad material has a sheet-like and rectangular or substantially rectangular shape in which notch(es) extending from the edge to the inside and capable of deforming a planar shape conforming to the shape of an sticking portion is (are) formed.

8. The pad material according to claim 7, wherein the notch is in a slit-shape having a width of 1 to 5 mm and a length of 10 to 40 mm, or a substantially trigonal shape having a maximum width of 1 to 5 mm and a length of 10 to 40 mm.

9. The pad material according to claim 3 or 7, wherein a heat dissipation promoting portion for conducting heat dissipation by increasing the area of contact with external area is formed at the surface or the rear face.

10. The pad material according to claim 9, wherein the heat dissipation promoting portion is a heat dissipating through hole from the surface to the rear face.

11. The pad material according to claim 9, wherein the heat dissipation promoting portion is a heat dissipating concave part or groove formed at the surface or the rear face.

12. The pad material according to claim 9, wherein the heat dissipation promoting portion is a heat dissipating through hole from one side edge to the other side edge of the sheet.

13. The pad material according to any one of claims 3, 7 and 9, wherein sucking concave part(s) is (are) formed on the surface or the rear face.

14. The pad material according to any one of claims 3, 7, 9 and 13, wherein the thickness at the periphery of the sheet is reduced toward the end edge at least a portion thereof.

15. The pad material according to claim 14, wherein a reinforcing rib is formed at the end edge of the sheet.

16. The pad material according to any one of claims 3, 7, 9, 13 and 14, wherein the pad material is sheet-like and curved smoothly to the inside at least at a portion of the circumferential edge thereof, and has a concave recess for enhancing the adhesion of the entire circumferential periphery of the sheet to the curved surface when applied to said curved surface.

17. The pad material according to claim 16, wherein the sheet has an oval or elliptic shape as a whole and one concaved recess is formed to the direction of the minor axis of the sheet.

18. The pad material according to claim 16, wherein the sheet has an oval or elliptic shape as a whole and two concaved recesses are formed opposite to each other in the direction of the minor axis of the sheet.

19. The pad material according to claim 16, wherein the sheet has a substantially polygonal shape as a whole and a concave recesse is formed at each of the sides of the sheet.

20. The pad material according to any one of claims 16 to 19, wherein the thickness of the sheet is 0.5 to 5 mm, the longitudinal longest portion is 50 to 350 mm, the lateral longest portion is 50 to 350 mm, the depth of the concave recesses is 2 to 100 mm, the width of the concave recess is 10 to 150 mm.

21. The pad material according to any one of claims 3 to 15, wherein the thickness of the sheet is 0.5 to 5 mm, the longitudinal longest portion is 20 to 250 mm, the lateral longest portion is 50 to 200 mm.

22. The pad material according to any one of claims 3 to 21, wherein fibers which are hydrophilic at least on the surface are dispersed inside the topical gel composition.

23. The pad material according to any one of claims 3 to 22, wherein a support is located on the surface or the rear face of the sheet.

24. The pad material according to any one of claims 3 to 23, wherein at least one of aroma oil and pharmacologically active substance is contained in the topical gel composition.

25. The pad material according to any one of claims 3 to 24, which is used as a cooling sheet.

26. The pad material according to any one of claims 22 to 24, which is used as a cosmetic sheet for face, lip or vicinity of eyes.

27. The pad material according to any one of claims 3 to 24, which is used as a medical sheet for limbs, shoulders, joints, backs, insect stings and pimples.

28. The gel cake substrate in which the topical gel composition according to claim 1 is absorbed in open cellular body.

29. The gel cake substrate according to claim 28, wherein at least one of aroma oil and pharmacologically active substance is contained in the topical gel composition.

30. A blister vessel-filled pad material, wherein a solution of a topical gel composition formed of water soluble polymer having self-shape retainability, 30 to 95% by weight of water content, and 65 to 99% of a compressive deformation rate expressed by the maximum deformation rate till a partial fracture of the gel structure when the gel composition is compressed at a predetermined speed by using a plunger capable of pressing the gel composition at a constant speed is filled in a blister vessel having a predetermined shape and molded into a sheet-like shape conforming to the shape of the blister vessel.

31. A blister vessel-packed pad material, wherein a topical gel composition formed of water soluble polymer having self-shape retainability, 30 to 95% by weight of water content, and 65 to 99% of a compressive deformation rate expressed by the maximum deformation rate till a partial fracture of the gel structure when the gel composition is compressed at a predetermined speed by using a plunger capable of pressing the gel composition at a constant speed is molded into a sheet-like shape, cut into a predetermined shape and packed in the blister vessel.

32. The blister vessel-filled or packed pad material according to claim 30 or 31, wherein a support is located on the surface or the rear face of the sheet.

33. The blister vessel-filled or packed pad material according to any one of claims 30 to 32, wherein the concave part of the blister vessel has a shape of a face or a body of an animal, or a shape of a face or a body of a character.
